# EUROPEAN PATENT APPLICATION

(11) **EP 4 585 203 A1**
(43) Date of publication of application: **16.07.2025**
(21) Application number: 23862832.5
(22) Date of filing: 01.08.2023
(51) Int. Cl.: A61K 8/34, A61K 8/14, A61K 8/55, A61Q 1/00, A61Q 19/00

(54) **METHOD FOR PRODUCING COMPOSITION CONTAINING LIPID MEMBRANE STRUCTURE**

(30) Priority: 05.09.2022 JP 2022140891
(71) Applicant: T. Hasegawa Co., Ltd., Tokyo 103-8431 (JP)
(72) Inventor: OCHI, Takao, Tokyo 103-8431 (JP)
(74) Representative: Hasegawa, Kan
(86) International application number: PCT/JP2023/028140
(87) International publication number: WO 2024/053289

(57) **Abstract**

According to the present disclosure, provided is a means that can control the particle diameter of a lipid membrane structure by a simple method when producing the lipid membrane structure. The present disclosure is a method for producing a lipid membrane structure-containing composition including: (A) a hydrogenated phospholipid having an acid value of 5 mg KOH/g or more; (B) a compound represented by formula 1; and (C) water. The method comprises a dispersing step of mixing the component (A), the component (B), and the component (C) to obtain a dispersion in which a lipid membrane structure having a particle diameter of 10 nm or more and 200 nm or less is spontaneously formed, wherein the dispersing step includes determining the concentration of the component (A) in the dispersion depending on the target particle diameter of the lipid membrane structure included in the lipid membrane structure-containing composition.

## Description

### Technical Field

The present invention relates to a method for producing a lipid membrane structure-containing composition.

### Background Art

Lipid membrane structures such as liposomes and bicelles have skin-caring effects such as a moisturizing effect and can encapsulate active ingredients. Accordingly, they are useful in application to cosmetic products and external preparations for the skin. As the raw materials of the lipid membrane structures for the above-mentioned application, phospholipids are mainly used because they are naturally-derived and have high safety. In particular, hydrogenated phospholipids, which is formed by adding hydrogen atoms to unsaturated carbon bonds of phospholipids, are preferably used because they are not easily degraded by oxidation.

Up to now, technology for forming lipid membrane structures by using hydrogenated phospholipids has been studied. For example, Japanese Patent Laid-Open No. 2011-32230 discloses that a liposome with high flexibility of its lipid membrane and storage stability can be obtained by preparing a composition through mixing a hydrogenated phospholipid with a ceramide, a branched alcohol, and others and dispersing it in an excessively large amount of water. National Publication of International Patent Application No. 2012-504620 (corresponding to the specification of WO 2010/039490) discloses that a fine monolayer liposome is obtained by mixing an oil-soluble composition containing a hydrogenated phospholipid, a glycol compound, and a lipophilic compound with a water-soluble composition to form a multi-layer liposome and then subjecting it to a conversion process.

### Summary of Invention

### Technical Problem

In application to cosmetic products and external preparations for the skin, a fine lipid membrane structure (for example, of a particle diameter of 200 nm or less) is required, in view of the ease of its permeation through the skin, exhibition of effects of the encapsulated active ingredients, storage stability, and so on. However, since hydrogenated phospholipids have high phase transition temperatures compared to not-hydrogenated phospholipids, it is difficult to form fine lipid membrane structures. Actually, in technology of forming lipid membrane structures by using hydrogenated phospholipids as described in Patent Literatures 1 and 2, it is necessary to use a refinement means such as a microfluidizer in order to form a fine lipid membrane structure (for example, of a particle diameter of 200 nm or less).

The lipid membrane structure preferably has a particle diameter adjusted according to the application. For example, as the particle diameter is smaller, the feel of the lipid membrane structure-containing composition when applied is lighter, the feel of its permeation through the skin is greater, and the appearance is closer to transparent. As the particle diameter is larger, the feel of the lipid membrane structure-containing composition when applied is heavier, the feel of its permeation through the skin is milder, and the appearance is closer to translucent or cloudy. Accordingly, it is required to design the particle diameter according to the intended feel and appearance. When the appearance is made to be translucent for the purpose of imparting high-quality appearance, there is a demand for technology that can appropriately adjust the particle diameter from the point of quality control, since the translucent degree varies depending on the particle diameter. For refinement means that are used industrially, such as a microfluidizer, it is difficult to control the particle diameter, and excessive investigation of conditions such as pressure and the number of passes is required. When the formulation of a composition and the conditions of an apparatus are not appropriately combined in investigation, there is a tendency to obtain a lipid membrane structure having a broad distribution of the particle diameter in terms of the dispersion uniformity. Even in a refinement means that is used in a small producing scale (for example, an ultrasonic treatment process or an extrusion process), the control of particle diameter is difficult, or although the particle diameter can be controlled, the operation is complicated. Accordingly, it has been difficult to obtain easily a liposome having a specific particle diameter.

Accordingly, it is an object of the present invention to provide a means that can control the particle diameter of a lipid membrane structure when producing the lipid membrane structure by a simple method.

### Solution to Problem

The present inventor has intensively studied in view of the above problem and, as a result, found that in a method for producing a lipid membrane structure-containing composition including: (A) a hydrogenated phospholipid having an acid value of 5 mg KOH/g or more; (B) a compound represented by the following formula 1: 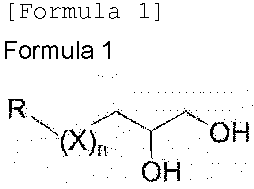 wherein R is a substituted or unsubstituted alkyl group having 2 to 6 carbon atoms or a substituted or unsubstituted cycloalkyl group having 3 to 6 carbon atoms, X is -O-, -C(=O)O-, or -O-C(=O)-, and n is 0 or 1); and (C) water, the concentration of the component (A) in a dispersion obtained by mixing these components exhibits a strong positive correlation with the particle diameter of a lipid membrane structure included in the lipid membrane structure-containing composition produced by the producing method (the particle diameter increases as the concentration increases) (hereinafter, this correlation is referred to as "the relationship of the present invention"). Based on this finding, it has been further found that the above problem is solved by controlling the concentration of the component (A) in the dispersion depending on the target particle diameter of the lipid membrane structure when obtaining the dispersion, and the present invention has been accomplished.

Specifically, an aspect of the present invention is a method for producing a lipid membrane structure-containing composition including the component (A), the component (B), and the component (C), the method comprising a dispersing step of mixing the component (A), the component (B), and the component (C) to obtain dispersion in which a lipid membrane structure having a particle diameter of 10 nm or more and 200 nm or less is spontaneously formed, wherein the dispersing step includes determining the concentration of the component (A) in the dispersion depending on the target particle diameter of the lipid membrane structure included in the lipid membrane structure-containing composition.

### Brief Description of Drawing

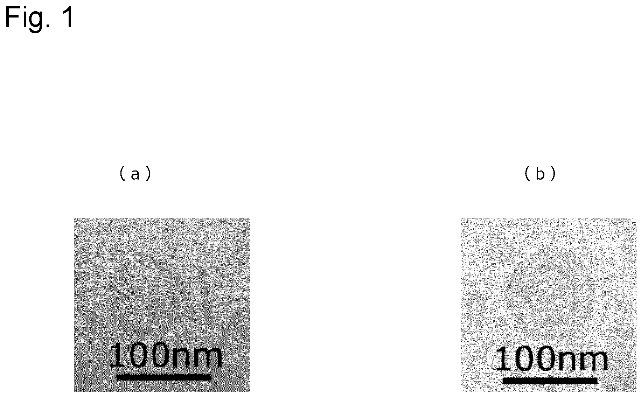

[Figure 1] Figure 1 is images of the lipid membrane structures of Examples photographed by cryo-electron microscopy (Cryo-TEM).

### Description of Embodiments

The present invention will now be described in detail. The present invention is not limited to the following embodiments only and can be variously modified within the scope of claims. The embodiments described in this specification can be combined in any manner to form other embodiments.

Throughout this specification, the expression in the singular form should be understood to include the concept of the plural form thereof, unless otherwise specified. Accordingly, singular articles (for example, "a", "an", and "the" in English) should be understood to include the concept of the plural form thereof, unless otherwise specified. The terms used in this specification should be understood to be used as the meaning commonly used in the art, unless otherwise specified. Accordingly, all technical and scientific terms used in this specification have the same meanings as commonly understood by a person skilled in the art to which the present invention pertains, unless otherwise specified. When conflict occurs, this specification (including definitions) takes precedence.

In this specification, "X to Y" is used to intend to include the numbers (X and Y) written before and after "to" as the lower limit and the upper limit and means "X or more and Y or less". Concentration and % mean concentration on mass basis and mass%, respectively, unless otherwise specified, and a ratio means a mass ratio, unless otherwise specified. Procedures and measurements of physical properties and so on are performed under conditions of room temperature (20°C to 25°C)/relative humidity of 40% to 50% RH, unless otherwise specified. "A and/or B" means encompassing each of A and B and a combination thereof.

An aspect of the present invention is a method for producing a lipid membrane structure-containing composition including: (A) a hydrogenated phospholipid having an acid value of 5 mg KOH/g or more; (B) a compound represented by the following formula 1: 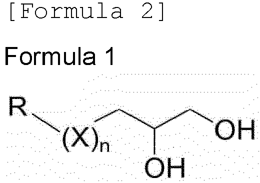 wherein R is a substituted or unsubstituted alkyl group having 2 to 6 carbon atoms or a substituted or unsubstituted cycloalkyl group having 3 to 6 carbon atoms, X is -O-, -C(=O)O-, or -O-C(=O)-, and n is 0 or 1); and (C) water, the method comprising a dispersing step of mixing the component (A), the component (B), and the component (C) to obtain dispersion in which a lipid membrane structure having a particle diameter of 10 nm or more and 200 nm or less is spontaneously formed, wherein the dispersing step includes determining the concentration of the component (A) in the dispersion depending on the target particle diameter of the lipid membrane structure included in the lipid membrane structure-containing composition. According to such a producing method of the present invention, the particle diameter of a lipid membrane structure can be controlled in a simple manner when producing the lipid membrane structure.

According to the lipid membrane structure-containing composition of the present invention, it is possible to form a fine liquid membrane structure having a target particle diameter (specifically, a particle diameter of 10 nm or more and 200 nm or less), without requiring a refinement means such as a microfluidizer.

As described above, a lipid membrane structure such as a liposome or a bicelle is useful in the application to cosmetic products and external preparations for the skin. In the above application, a fine lipid membrane structure (for example, of a particle diameter of 200 nm or less) is required in view of the ease of permeation through the skin, expression of effects of the encapsulated active ingredients, storage stability, and so on. Since the desirable particle diameter of a lipid membrane structure varies depending on which point of view in the above is intended, it is required to control the particle diameter according to the application.

As the raw materials of the lipid membrane structures for the above-mentioned application, phospholipids are mainly used because they are naturally-derived and have high safety. In particular, hydrogenated phospholipids are preferably used because they are not easily degraded by oxidation. However, hydrogenated phospholipids have high phase transition temperatures compared to not-hydrogenated phospholipids, and the physical properties of both largely differ from each other. Consequently, in the technology described in Patent Literatures 1 and 2, it is difficult to form easily a fine lipid membrane structure (for example, of a particle diameter of 200 nm or less) by using a hydrogenated phospholipid having a high phase transition temperature, and therefore, a lipid membrane structure having a target particle diameter cannot be obtained.

Accordingly, the present inventor has intensively studied on means for forming easily fine lipid membrane structures by using hydrogenated phospholipids. As a result, it has been found surprisingly that when (A) a hydrogenated phospholipid having an acid value of 5 mg KOH/g or more, (B) a compound represented by the above formula 1 (hereinafter, also referred to as "polyol compound"), and (C) water are mixed to obtain a dispersion in producing a lipid membrane structure-containing composition, a fine lipid membrane structure having a target particle diameter (specifically, a particle diameter of 10 nm or more and 200 nm or less) is formed by changing the concentration of the component (A) in the dispersion depending on the target particle diameter of the lipid membrane structure (that is, there is the relationship of the present invention), without requiring a refinement means. In the present invention, the target particle diameter of a lipid membrane structure is within a range of 10 nm or more and 200 nm or less.

### <Method for producing lipid membrane structure-containing composition>

The method for producing a lipid membrane structure-containing composition according to an aspect of the present invention comprises a dispersing step of mixing (A) a hydrogenated phospholipid having an acid value of 5 mg KOH/g or more, (B) a compound represented by the following formula 1: 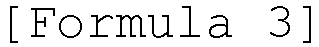 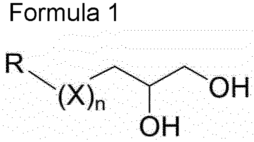 wherein R is a substituted or unsubstituted alkyl group having 2 to 6 carbon atoms or a substituted or unsubstituted cycloalkyl group having 3 to 6 carbon atoms, X is -O-, -C(=O)O-, or -O-C(=O)-, and n is 0 or 1), and (C) water to obtain a dispersion in which a lipid membrane structure having a particle diameter of 10 nm or more and 200 nm or less is spontaneously formed, wherein the dispersing step further includes determining the concentration of the component (A) in the dispersion depending on the target particle diameter of the lipid membrane structure included in the lipid membrane structure-containing composition.

Namely, in a method for producing a lipid membrane structure-containing composition according to an aspect of the present invention, a target particle diameter of the lipid membrane structure to be produced is set, which is considered in light of the relationship of the present invention to determine the concentration of the component (A) in the dispersion prepared in the dispersing step. The lipid membrane structure produced by the producing method according to the present invention has a particle diameter of 10 nm or more and 200 nm or less.

Before describing the producing method of the present invention, the lipid membrane structure will first be described below.

### [Lipid membrane structure]

The form of the lipid membrane structure is not particularly limited as long as it has a lamellar (lipid bilayer) structure in which lipid molecules are arranged with their hydrophilic groups facing outward and their hydrophobic groups facing inward, and examples thereof include a liposome, a bicelle, and α gel. The lipid membrane structure may be a monolayer lamellar structure, which is called unilamella or single lamella, or may be a multilayer lamellar structure of several layers (2 to 10 layers), which is called oligolamella, or may be a multilayer lamellar structure of more layers, or may be a mixture thereof.

Whether a lipid membrane structure is a monolayer lamellar structure (hereinafter, also referred to as "monolayer") or a multilayer lamellar structure (hereinafter, also referred to as "multilayer") can be verified using, for example, cryo-electron microscopy (Cryo-TEM).

In an embodiment of the present invention, the lipid membrane structure is preferably a monolayer lamellar liposome. A monolayer lamellar liposome has a large internal aqueous phase volume while having a fine particle diameter and is therefore a monolayer lamellar structure having excellent efficiency in encapsulating water-soluble ingredient and ease of permeation. Therefore, it is useful in cosmetics and external preparations for the skin containing water-soluble cosmetic ingredients. In an embodiment of the present invention, the lipid membrane structure is preferably a bicelle. A bicelle is a fine discotic monolayer lamellar structure with a thickness of 3 to 10 nm and a diameter of 15 to 100 nm and has excellent efficiency in encapsulating lipophilic ingredient and ease of permeation. Therefore, it is useful in cosmetics and external preparations for the skin containing lipophilic cosmetic ingredients.

In an embodiment of the present invention, the lipid membrane structure is preferably a multilayer lamellar liposome. A multilayer lamellar liposome has a large lamellar volume while having a fine particle diameter and can thus incorporate a lipophilic ingredient in a lamella; therefore, it has excellent efficiency in encapsulating lipophilic ingredient and ease of permeation. Therefore, it is useful in cosmetics and external preparations for the skin containing lipophilic cosmetic ingredients.

In the lipid membrane structure-containing composition according to the present invention, only one of a monolayer lamellar liposome, a bicelle, or a multilayer lamellar liposome may be present, or two or more selected from the group consisting of a multilayer lamellar liposome, a monolayer lamellar liposome, and a bicelle may be present as a mixture.

In the present invention, the lipid membrane structure has a particle diameter of 10 nm or more and 200 nm or less. The particle diameter of the lipid membrane structure is preferably 20 nm or more and 190 nm or less, more preferably 30 nm or more and 180 nm or less, further preferably 40 nm or more and 170 nm or less, particularly preferably 50 nm or more and 160 nm or less, and most preferably 50 nm or more and 150 nm or less. The upper limit of the particle diameter of the lipid membrane structure is not particularly limited, and the particle diameter is, for example, 200 nm or less, 190 nm or less, 180 nm or less, 170 nm or less, 160 nm or less, 150 nm or less, 140 nm or less, 135 nm or less, or 130 nm or less, in view of dispersibility, storage stability, and ease of permeation through the skin. The lower limit of the particle diameter of the lipid membrane structure is not particularly limited, and the particle diameter is, for example, 10 nm or more, 20 nm or more, 30 nm or more, 40 nm or more, or 50 nm or more, in view of lipid membrane structure-forming efficiency.

The upper limit of the polydispersity index (PDI) of the lipid membrane structure is not particularly limited, and the PDI is, for example, 0.80 or less, 0.70 or less, 0.60 or less, 0.50 or less, 0.40 or less, 0.30 or less, 0.25 or less, or 0.20 or less, in view of dispersibility, storage stability, and ease of permeation through the skin. The lower limit of the polydispersity index (PDI) of the lipid membrane structure is not particularly limited, and the PDI is, for example, 0.01 or more.

In this specification, the particle diameter of a lipid membrane structure means an average hydrodynamic diameter. As the particle diameter (average hydrodynamic diameter) and polydispersity index (PDI) of a lipid membrane structure, a harmonic mean diameter (Z-Average) and polydispersity index (PDI) based on scattered light intensity by cumulant analysis using a dynamic light scattering measuring apparatus (produced by Malvern Instruments, Zetasizer Nano ZSP) are used. The definitions of the particle diameter and the polydispersity index used here conform to the description in "JIS Z8828: 2019 Particle size analysis - Dynamic light scattering".

The lipid membrane structure-containing composition of the present invention includes the above-described lipid membrane structure. In this specification, if the particle diameter of a lipid membrane structure-containing composition can be measured using a dynamic light scattering measuring apparatus, more specifically, Zetasizer Nano ZSP (produced by Malvern Instruments), it is determined that the lipid membrane structure is formed.

The lipid membrane structure to be included in the lipid membrane structure-containing composition of the present invention is formed by dispersing a component for forming a lipid membrane structure in the component (C) (water). In the present invention, a component for forming a lipid membrane structure is dispersed in the component (C) in the dispersing step to form a lipid membrane structure. Accordingly, the dispersion obtained by the dispersing step is a solution in which a lipid membrane structure has been formed. Specifically, in the present invention, the dispersion obtained by the dispersing step is of a lipid membrane structure having a particle diameter of 10 nm or more and 200 nm or less spontaneously formed.

Here, the lipid membrane structure-containing composition of the present invention encompasses (i) a dispersion obtained by the dispersing step and (ii) a dispersion of which concentration has been further adjusted with the component (C) after the dispersing step. Accordingly, in the case of (i), the dispersion of the present invention is directly a lipid membrane structure-containing composition. In the case of (ii), the dispersion of the present invention is further subjected to adjustment of concentration with the component (C) and thus becomes a lipid membrane structure-containing composition. Accordingly, in the case of (i), a lipid membrane structure-containing composition is obtained by the dispersing step, and in the case of (ii), a lipid membrane structure-containing composition is obtained from a dispersion through a concentration-adjusting step described later. The component (C) in which the component for forming the lipid membrane structure is dispersed may be used together with a component other than the component (C), for example, may be used together with components (D) to (F) described later or components described in the paragraph of [Additional component] described later or a mixture thereof. The component (C) in a system in which the lipid membrane structure is present (that is, component (C-2) and component (C-3) described later) may be referred to as an "aqueous phase". For example, the component (C-2) and component (C-3) in which a component for forming the lipid membrane structure has been dispersed serve as an "aqueous phase" for the component for forming the lipid membrane structure.

In the method for forming a lipid membrane structure-containing composition of the present invention, the component (C) is used as a component to be added in a mixing step and a concentration-adjusting step described later, in addition to the dispersing step. Accordingly, hereinafter, the component (C) to be added for mixing in the mixing step is referred to as "component (C-1)" or "water (for source agent)", the component (C) to be added for dispersing in the dispersing step is referred to as "component (C-2)" or "water (for dispersion)", and the component (C) to be added for adjustment of the concentration in the concentration-adjusting step is referred to as "component (C-3)" or "water (for adjustment of concentration)". The amount of the component (C) included in the lipid membrane structure-containing composition is the sum of the amounts of the component (C-1) added for mixing in the mixing step, the component (C-2) added for dispersing in the dispersing step, and the component (C-3) added for adjustment of the concentration in the concentration-adjusting step described later. In the method for producing a lipid membrane structure-containing composition of the present invention, the mixing step and the concentration-adjusting step are steps that are performed if needed.

A preferable embodiment of the producing method according to an aspect of the present invention will now be described.

### [Dispersing step]

The dispersing step is a step of mixing a component (A), a component (B), and a component (C-2) to obtain a dispersion in which a lipid membrane structure having a particle diameter of 10 nm or more and 200 nm or less is spontaneously formed. On this occasion, in the dispersing step, the concentration of the component (A) in the dispersion is determined depending on the target particle diameter of the lipid membrane structure included in the lipid membrane structure-containing composition, on the basis of the relationship of the present invention. Specifically, in order to produce a lipid membrane structure with a larger particle diameter, the concentration of the component (A) in the dispersion can be increased. The producing method according to this aspect may further include determining a correlation between the concentration of the component (A) in the dispersion and the particle diameter of the lipid membrane structure included in the lipid membrane structure-containing composition (the relationship of the present invention). This correlation in a composition of an intended lipid membrane structure-containing composition can be determined by performing preliminary experiments for obtaining lipid membrane structure-containing compositions having various particle diameters by changing the concentration of the component (A) in the dispersion and executing known regression analysis such as an ordinary least square (OLS) method. Here, the specific expression of "the relationship of the present invention" determined by known regression analysis is not particularly limited, but may be, for example, an approximate straight line (linear regression) or an approximate curve (polynomial approximation). Furthermore, the degree of the approximate curve (polynomial approximation) is not particularly limited, but is preferably 2nd to 5th order and more preferably 3rd or 4th order. These approximate straight line and approximate curve can be acquired using the function of "linear approximation" and "polynomial approximation" in the "approximate curve formatting" of Microsoft Excel (produced by Microsoft Corporation). When "the relationship of the present invention" that has been separately obtained by using such a method is available, the producing method according to this embodiment can be carried out using it. In this case, the producing method according to this embodiment does not include the determination of "the relationship of the present invention". The target particle diameter of a lipid membrane structure can be appropriately set in the range of the particle diameter stated in the section of [Lipid membrane structure]. For example, the target particle diameter of the lipid membrane structure is 10 nm or more and 200 nm or less.

The target particle diameter of a lipid membrane structure is determined, and then the concentration of the component (A) in the dispersion is determined depending on the particle diameter (in light of "the relationship of the present invention"). The concentration of the component (A) determined depending on the target particle diameter of the lipid membrane structure is, for example, preferably 0.01 to 15 mass%, more preferably 0.05 to 15 mass%, further preferably 0.1 to 12 mass%, further more preferably 0.2 to 10 mass%, particularly preferably 0.3 to 7.5 mass%, and most preferably 0.5 to 5 mass%, relative to the dispersion (total mass: 100 mass%). For the lower limit, the concentration of the component (A) determined depending on the target particle diameter of the lipid membrane structure is, for example, 0.01 mass% or more, 0.02 mass% or more, 0.1 mass% or more, 0.2 mass% or more, 0.3 mass% or more, 0.4 mass% or more, 0.5 mass% or more, 0.6 mass% or more, 0.7 mass% or more, 0.8 mass% or more, 0.9 mass% or more, 1.0 mass% or more, 1.1 mass% or more, 1.2 mass% or more, 1.3 mass% or more, 1.4 mass% or more, 1.5 mass% or more, 1.6 mass% or more, 1.7 mass% or more, 1.8 mass% or more, 1.9 mass% or more, or 2.0 mass% or more, relative to the dispersion (total mass: 100 mass%). For the upper limit in this case, the concentration of the component (A) is 15 mass% or less, 14 mass% or less, 13 mass% or less, 12 mass% or less, 11 mass% or less, 10 mass% or less, 9.5 mass% or less, 9.0 mass% or less, 8.5 mass% or less, 8.0 mass% or less, 7.5 mass% or less, 7.0 mass% or less, 6.5 mass% or less, 6.0 mass% or less, 5.5 mass% or less, or 5.0 mass% or less, relative to the dispersion (total mass: 100 mass%). In an embodiment, the concentration of the component (A) determined depending on the target particle diameter of the lipid membrane structure in the dispersion is 0.05 mass% or more and less than 4.5 mass%. The particle diameter of the resulting lipid membrane structure can be controlled by adjusting the concentration of the component (A) in the above range. Thus, the present invention has been accomplished by finding that the particle diameter of the lipid membrane structure included in a lipid membrane structure-containing composition can be controlled by producing the lipid membrane structure-containing composition through a process in which the concentration of the component (A) is within the above range.

Namely, in producing a lipid membrane structure-containing composition, the concentration of the compound (A) in the dispersion obtained in the dispersing step varies depending on the target particle diameter of a lipid membrane structure (in light of the relationship of the present invention). Consequently, a lipid membrane structure with a target particle diameter is formed in the finally obtained lipid membrane structure-containing composition. According to such an aspect, the particle diameter of the lipid membrane structure in the lipid membrane structure-containing composition can be controlled, and thus, the particle diameter can be advantageously set according to the purpose.

Here, the present inventor has found that according to the producing method according to an aspect of the present invention, the structure of the lipid membrane structure included in the lipid membrane structure-containing composition also can be controlled. In Examples, when the concentration of the component (A) in the dispersing step of the lipid membrane structure-containing composition is 2 mass% or less relative to the dispersion (total mass: 100 mass%), a lipid membrane structure having a monolayer lamellar liposome structure was observed (e.g., Examples 1-1 and 1-2). In contrast, when the concentration of the component (A) in the dispersing step of the lipid membrane structure-containing composition is 3 mass% or more relative to the dispersion (total mass: 100 mass%), a lipid membrane structure having a multilayer lamellar liposome structure was observed (e.g., Examples 1-3 to 1-5). In other words, it has been found that according to the producing method of the present invention, the structure of the lipid membrane structure can be controlled by adjusting the concentration of the component (A) in light of the relationship of the present invention.

A producing method according to an aspect of the present invention is preferably a method in which a lipid membrane structure is formed at a concentration of the component (A) higher than the target concentration of the component (A) in a lipid membrane structure-containing composition to be obtained as a final product and then a component (C-3) is added down to the target concentration of the component (A) to obtain the lipid membrane structure-containing composition. In other words, an embodiment of the present invention may further include a concentration-adjusting step for mixing the dispersion with a component (C-3) (water) after the dispersing step. According to such an embodiment, a lipid membrane structure is formed in a system having a high concentration of the component (A), and the system is then diluted. Such a process can vary the particle diameter of the lipid membrane structure in the lipid membrane structure-containing composition even between cases where the formulation (the contents of respective components, in particular, the concentration of the component (A)) of the lipid membrane structure-containing composition is the same. Namely, the particle diameter of the lipid membrane structure can be controlled in lipid membrane structure-containing compositions of the same formulation, and therefore, the particle diameter can be set according to the purpose.

The dispersion obtained by the dispersing step includes the component (A), the component (B), and the component (C) (component (C-2)) and also includes components (D) to (F) and a component described later in the section [Additional component], as needed. These components constituting the dispersion will be described. Even when the dispersion includes a component other than the components (A) to (C-2), the relationship of the present invention is present according to the formulation. Accordingly, even in such a case, the producing method according to this aspect can be carried out on the basis of the relationship of the present invention in the same manner as in above.

### [Component (A)]

The dispersion according to the present invention includes a hydrogenated phospholipid having an acid value of 5 mg KOH/g or more as the component (A). The component (A) may be one or more hydrogenated phospholipids having an acid value of 5 mg KOH/g or more or may be a mixture of one or more hydrogenated phospholipids having an acid value of less than 5 mg KOH/g and one or more hydrogenated phospholipids having an acid value of 5 mg KOH/g or more combined with each other and adjusted to an acid value of 5 mg KOH/g or more.

The hydrogenated phospholipid can be obtained by adding a hydrogen atom to an unsaturated carbon bond of a phospholipid by a conventionally known method. The origin of the phospholipid is not particularly limited, but lecithin is particularly preferable because it is naturally-derived and can be suitably used in cosmetic products and external preparations for the skin. Accordingly, in an embodiment of the present invention, the component (A) is hydrogenated lecithin. Lecithin may be derived from soybeans, egg yolks, rapeseed, sunflower, or corn, for example, and is preferably derived from a plant such as soybeans, rapeseed, sunflower, and corn, and more preferably from soybeans because of availability and stability in terms of quality.

The acid value of the component (A) is 5 mg KOH/g or more. If a hydrogenated phospholipid having an acid value of less than 5 mg KOH/g is used, a fine lipid membrane structure cannot be formed even when it is combined with a component (B). In view of obtaining a finer lipid membrane structure, the acid value of the component (A) is preferably 12 mg KOH/g or more, more preferably 15 mg KOH/g or more, further preferably 17 mg KOH/g or more, further more preferably 20 mg KOH/g or more, and particularly preferably 22 mg KOH/g or more. The acid value of the component (A) is, for example, 6 mg KOH/g or more, 7 mg KOH/g or more, 8 mg KOH/g or more, 9 mg KOH/g or more, 10 mg KOH/g or more, 11 mg KOH/g or more, 12 mg KOH/g or more, 13 mg KOH/g or more, 14 mg KOH/g or more, 15 mg KOH/g or more, 16 mg KOH/g or more, 17 mg KOH/g or more, 18 mg KOH/g or more, 19 mg KOH/g or more, 20 mg KOH/g or more, 21 mg KOH/g or more, 22 mg KOH/g or more, 23 mg KOH/g or more, 24 mg KOH/g or more, 25 mg KOH/g or more, 26 mg KOH/g or more, or 27 mg KOH/g or more. The acid value of the component (A) is, for example, 70 mg KOH/g or less, 60 mg KOH/g or less, 50 mg KOH/g or less, or 40 mg KOH/g or less, but not particularly limited thereto. A hydrogenated phospholipid having an intended acid value can be obtained by appropriately selecting the type of the phospholipid. As the acid value of the component (A), a value measured in accordance with "Japanese Standards of Quasi-drug Ingredients 2021, General Test Method, 28. Acid Value Measurement Method" is used.

As the component (A), either a synthetic product or a commercially available product may be used. Examples of the commercially available product include EMALEX (registered trademark) SLP produced by Nihon Emulsion Co., Ltd. and SLP White H produced by Tsuji Oil Mills Co., Ltd. These products may be used singly or in combination of two or more.

The content of the component (A) in the dispersion according to the present invention is the above-described "concentration of the component (A) determined depending on the target particle diameter of the lipid membrane structure". Accordingly, the concentration of the component (A) determined depending on the target particle diameter of the lipid membrane structure described above is applied.

### [Component (B)]

The dispersion according to the present invention includes a compound represented by the formula 1 below as the component (B). The component (B) may consist of one compound or two or more compounds.

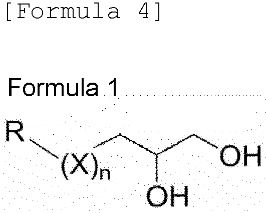

In the formula 1 above, R is a substituted or unsubstituted alkyl group having 2 to 6 carbon atoms or a substituted or unsubstituted cycloalkyl group having 3 to 6 carbon atoms.

The alkyl group having 2 to 6 carbon atoms may be either linear or branched. Examples of the alkyl group having 2 to 6 carbon atoms include an ethyl group, an n-propyl group, an isopropyl group, an n-butyl group, an isobutyl group, a sec-butyl group, a tert-butyl group, an n-pentyl group, an iso-amyl group, a tert-pentyl group, a neopentyl group, an n-hexyl group, a 3-methylpentan-2-yl group, a 3-methylpentan-3-yl group, a 4-methylpentyl group, a 4-methylpentan-2-yl group, a 1,3-dimethylbutyl group, a 3,3-dimethylbutyl group, and a 3,3-dimethylbutan-2-yl group. The alkyl group having 2 to 6 carbon atoms is preferably linear. That is, the alkyl group having 2 to 6 carbon atoms is preferably a group selected from the group consisting of an ethyl group, an n-propyl group, an n-butyl group, an n-pentyl group, and an n-hexyl group.

Examples of the cycloalkyl group having 3 to 6 carbon atoms include a cyclopropyl group, a cyclobutyl group, a cyclopentyl group, and a cyclohexyl group, and a cyclohexyl group is preferable.

The substituent that may be present on the alkyl group having 2 to 6 carbon atoms or the cycloalkyl group having 3 to 6 carbon atoms is not particularly limited, as long as the effect of the present invention is exhibited. Examples of the substituent include a halogen atom, an acyl group, an alkyl group, an aryl group, an alkoxyl group, a nitro group, an amino group, and a cyano group. However, the alkyl group having 2 to 6 carbon atoms is not substituted with an alkyl group.

In the formula 1 above, X is -O-, -C(=O)O-, or -O-C(=O)- and is preferably -O-. In the formula 1 above, n is 0 or 1. In the formula 1, when X is -O- with n=1, the number of carbon atoms of the alkyl group as R is preferably 4 or more.

The component (B) is preferably, for example, at least one selected from the group consisting of 1,2-pentanediol, 1,2-hexanediol, 1,2-heptanediol, 1,2-octanediol, cyclohexyl glycerin, and hexyl glycerin.

In particular, when 1,2-hexanediol, 1,2-heptanediol, or hexyl glycerin is used as the component (B), a significantly fine lipid membrane structure can be formed. It is believed that on this occasion, the lipid membrane structure forms a bicelle. The bicelle has a discotic monolayer lamellar structure and is therefore excellent in the permeation through horny cell layer compared to a liposome having a similar particle diameter. Accordingly, in view of obtaining a lipid membrane structure with great permeation of a lipophilic component, it is preferable to use, as the component (B), at least one selected from the group consisting of 1,2-hexanediol, 1,2-heptanediol, and hexyl glycerin.

The component (B) may be either a synthetic product or a commercially available product.

The content of the component (B) in the dispersion according to the present invention is preferably greater than 0.01 mass% and 24 mass% or less, more preferably 0.02 mass% or more and 23 mass% or less, further preferably 0.05 mass% or more and 20 mass% or less, particularly preferably 0.1 mass% or more and 18 mass% or less, and most preferably 0.2 mass% or more and 15 mass% or less, relative to the dispersion (total mass: 100 mass%). For the lower limit, the content of the component (B) in the dispersion is preferably, for example, greater than 0.01 mass% relative to the dispersion (total mass: 100 mass%). For the lower limit, the content of the component (B) in the dispersion is 0.011 mass% or more, 0.02 mass% or more, 0.05 mass% or more, 0.1 mass% or more, 0.2 mass% or more, 0.3 mass% or more, 0.4 mass% or more, 0.5 mass% or more, 0.6 mass% or more, 0.7 mass% or more, 0.8 mass% or more, 0.9 mass% or more, 1.0 mass% or more, 1.1 mass% or more, 1.2 mass% or more, 1.3 mass% or more, 1.4 mass% or more, 1.5 mass% or more, 1.6 mass% or more, 1.7 mass% or more, 1.8 mass% or more, 1.9 mass% or more, or 2.0 mass% or more, relative to the dispersion (total mass: 100 mass%). For the upper limit, the content of the component (B) in the dispersion is preferably 24 mass% or less relative to the dispersion (total mass: 100 mass%). For the upper limit, the content of the component (B) in the dispersion is 23 mass% or less, 22 mass% or less, 21 mass% or less, 20 mass% or less, 19 mass% or less, 18 mass% or less, 17 mass% or less, 16 mass% or less, 15 mass% or less, 14 mass% or less, 13 mass% or less, 12 mass% or less, 11 mass% or less, 10 mass% or less, 9.5 mass% or less, 9.0 mass% or less, 8.5 mass% or less, 8.0 mass% or less, 7.5 mass% or less, 7.0 mass% or less, 6.5 mass% or less, 6.0 mass% or less, 5.5 mass% or less, 5.0 mass% or less, or 4.5 mass% or less.

In the dispersion according to the present invention, the content of the component (B) is preferably greater than 100 parts by mass, more preferably 110 parts by mass or more and 2000 parts by mass or less, further preferably 120 parts by mass or more and 1800 parts by mass or less, further more preferably 130 parts by mass or more and 1500 parts by mass or less, particularly preferably 140 parts by mass or more and 1200 parts by mass or less, and most preferably 150 parts by mass or more and 1000 parts by mass or less, per 100 parts by mass of the component (A). When the content of the component (B) is within the above range, the component (A) easily forms a lipid membrane structure more efficiently in the dispersion, and a lipid membrane structure with excellent dispersibility can be formed. Furthermore, in view of obtaining a finer lipid membrane structure, the content of the component (B) is, for example, 160 parts by mass or more, 170 parts by mass or more, 180 parts by mass or more, 190 parts by mass or more, 200 parts by mass or more, 210 parts by mass or more, 220 parts by mass or more, 230 parts by mass or more, 240 parts by mass or more, 250 parts by mass or more, 260 parts by mass or more, 270 parts by mass or more, 280 parts by mass or more, 290 parts by mass or more, 300 parts by mass or more, 310 parts by mass or more, 320 parts by mass or more, 330 parts by mass or more, 340 parts by mass or more, 350 parts by mass or more, 360 parts by mass or more, 370 parts by mass or more, 380 parts by mass or more, 390 parts by mass or more, or 400 parts by mass or more, per 100 parts by mass of the component (A). On the other hand, the upper limit of the content of the component (B) per 100 parts by mass of the component (A) is not particularly limited; however, even if it exceeds 2000 parts by mass, the solubility or dispersibility of the component (A) in the dispersion is not affected, and it is simply uneconomical. Consequently, the content of the component (B) is, for example, 2000 parts by mass or less, 1900 parts by mass or less, 1800 parts by mass or less, 1700 parts by mass or less, 1600 parts by mass or less, 1500 parts by mass or less, 1400 parts by mass or less, 1300 parts by mass or less, 1200 parts by mass or less, 1100 parts by mass or less, 1000 parts by mass or less, 900 parts by mass or less, 800 parts by mass or less, 700 parts by mass or less, 600 parts by mass or less, or 500 parts by mass or less, per 100 parts by mass of the component (A). Thus, according to a preferable embodiment of the present invention, the content of the component (B) is preferably 110 parts by mass or more and 2000 parts by mass or less per 100 parts by mass of the component (A).

### [Component (C-2))]

The dispersion according to the present invention includes water as the component (C-2). The component (C-2) has a role as a medium for dispersing a component that forms a lipid membrane structure and is a main component that forms an aqueous phase in the dispersion. In addition, the component (C-2) has a role in helping a component (D) (basic compound) and a component (E) (acidic compound), which will be described later, to be incorporated in the dispersion. Specifically, the component (D) and/or the component (E) can be dissolved in the component (C-2) in advance and then the resultant can be mixed with the component (A) and the component (B) to thereby easily incorporate the component (D) and/or the component (E) in the dispersion.

The component (C-2) is preferably water with fewer impurities, and for example, it is preferably those obtained by purifying ordinary water using a system of one or combination of two or more of ion exchange, distillation, reverse osmosis, ultrafiltration, and so on, such as purified water.

The content of the component (C-2) in the dispersion according to the present invention is preferably 50 mass% or more and 99.97 mass% or less, more preferably 60 mass% or more and 99.9 mass% or less, further preferably 60 mass% or more and 99.8 mass% or less, particularly preferably 65 mass% or more and 99.7 mass% or less, and most preferably 70 mass% or more and 99.6 mass% or less, relative to the dispersion (total mass: 100 mass%). For the lower limit, the content of the component (C-2) in the dispersion is, for example, 50 mass% or more, 55 mass% or more, 60 mass% or more, 65 mass% or more, 70 mass% or more, 75 mass% or more, 80 mass% or more, 85 mass% or more, or 90 mass% or more, relative to the dispersion (total mass: 100 mass%). For the upper limit, the content of the component (C-2) in the dispersion is, for example, 99.97 mass% or less, 99.9 mass% or less, 99.8 mass% or less, 99.7 mass% or less, or 99.6 mass% or less, relative to the dispersion (total mass: 100 mass%).

For the lower limit, the mass ratio of the component (B) to the component (C-2) ("component (B) : component (C-2)") in the dispersion according to the present invention is preferably greater than 0.01 : 99.99 and is 0.1 : 99.9 or more, 1 : 99 or more, 2 : 98 or more, or 3 : 97 or more. For the upper limit, the mass ratio of the component (B) to the component (C-2) in the dispersion is preferably less than 25 : 75 and is 24 : 76 or less, 20 : 80 or less, 16 : 84 or less, 12.5 : 87.5 or less, or 10 : 90 or less.

In the present invention, the mixture obtained by mixing the component (A), the component (B), and the component (C-2) (i.e., component (C)) in which a lipid membrane structure of a particle diameter of 10 nm or more and 200 nm or less is spontaneously formed is referred to as "dispersion", and the process in which the component (A), the component (B), and the component (C-2) are mixed to allow a lipid membrane structure of a particle diameter of 10 nm or more and 200 nm or less to be spontaneously formed is referred to as "dispersing step". For example, the component (A), the component (B), and the component (C-1) (i.e., component (C)) may be mixed in the mixing step described later, but even if a component (C-1) (i.e., component (C)) is present, there is no chance of "spontaneously forming a lipid membrane structure of a particle diameter of 10 nm or more and 200 nm or less" in this mixing step. The lipid membrane source agent obtained in the mixing step does not have a lipid membrane structure with a particle diameter of 10 nm or more and 200 nm or less. Accordingly, in order to determine whether it is a dispersion according to the present invention or not (for example, whether it is a dispersion or a lipid membrane source agent), the particle diameter may be measured to verify whether "a lipid membrane structure having a particle diameter of 10 nm or more and 200 nm or less" has been formed or not.

For example, one of methods for controlling the "spontaneous formation of a lipid membrane structure with a particle diameter of 10 nm or more and 200 nm or less" is to adjust amounts of the component (B) and the component (C-2) in the dispersion to a predetermined ratio. Specifically, when the mass ratio of the component (B) to the component (C-2) ("component (B) : component (C-2)") in the dispersion is greater than 0.01 : 99.98 and less than 25 : 75, a lipid membrane structure having a particle diameter of 10 nm or more and 200 nm or less is spontaneously formed by mixing the component (A), the component (B), and the component (C-2), so that a dispersion can be obtained.

### [Component (D)]

The dispersion according to the present invention may further contain a basic compound as a component (D), in addition to the component (A), component (B), and component (C-2). When a component (A) with a high acid value is used together with the component (D), a dispersion having highly excellent dispersibility of the lipid membrane structure can be obtained. The component (D) may consist of one compound or two or more compounds.

Examples of the component (D) include inorganic bases such as sodium hydroxide, potassium hydroxide, and ammonia; basic amino acids such as arginine, lysine, and histidine; and amine compounds such as ethanolamine, diethanolamine, triethanolamine, 2-amino-2-methyl-1,3-propanediol (AMPD), and 2-amino-2-hydroxymethyl-1,3-propanediol (tromethamine). In particular, arginine is preferable.

As the component (D), either a synthetic product or a commercially available product may be used.

The content of the component (D) in the dispersion according to the present invention is preferably 0.000005 mass% or more and 1.5 mass% or less, more preferably 0.00001 mass% or more and 1.5 mass% or less, further preferably 0.0001 mass% or more and 1.0 mass% or less, particularly preferably 0.001 mass% or more and 1.0 mass% or less, and most preferably 0.01 mass% or more and 1.0 mass% or less, relative to the dispersion (total mass: 100 mass%). For the lower limit, the content of the component (D) in the dispersion is 0.000005 mass% or more, 0.00001 mass% or more, 0.0001 mass% or more, 0.001 mass% or more, 0.01 mass% or more, or 0.02 mass% or more, relative to the dispersion (total mass: 100 mass%). For the upper limit, the content of the component (D) in the dispersion is preferably 1.5 mass% or less and more preferably 1.0 mass% or less, relative to the dispersion (total mass: 100 mass%).

For the lower limit, the content of the component (D) in the dispersion according to the present invention is preferably 0.05 parts by mass or more, and more preferably 0.1 parts by mass or more, per 100 parts by mass of the component (A). For the upper limit, the content of the component (D) is preferably 10 parts by mass or less, and more preferably 5 parts by mass or less.

### [Component (E)]

The dispersion according to the present invention may contain an acidic compound as a component (E), in addition to the component (A), component (B), and component (C-2). The dispersibility of the lipid membrane structure in the aqueous phase can be enhanced by the component (E) contained. The component (E) may consist of one compound or two or more compounds.

Examples of the component (E) include inorganic acids such as hydrochloric acid, sulfuric acid, nitric acid, phosphoric acid, and carbonic acid; and organic acids such as acetic acid, formic acid, propionic acid, butyric acid, citric acid, lactic acid, succinic acid, malic acid, tartaric acid, pyrrolidone carboxylic acid (PCA), gluconic acid, benzoic acid, ethylenediaminetetraacetic acid (EDTA), etidronic acid, pentetic acid, and phytic acid. In particular, in view of obtaining a dispersion highly excellent in dispersibility of the lipid membrane structure, an organic acid is preferable.

In particular, in view of forming a lipid membrane structure more efficiently and obtaining a dispersion with highly excellent dispersibility and in view of enhancing the storage stability of the dispersion and a lipid membrane structure-containing composition including the dispersion, the component (E) is preferably an organic acid having a chelating effect. The component (A), which is a main component for forming a lipid membrane structure, may have a property of easily bonding to metal ions. In this case, by adding an organic acid having a chelating effect as the component (E), metal ions in a dispersion are captured to easily form a lipid membrane structure more efficiently, so that the dispersion obtained can have highly excellent dispersibility of the lipid membrane structure, and also, the storage stability of the dispersion and a lipid membrane structure-containing composition including the dispersion can be enhanced. Namely, it is inferred that in the dispersion, a lipid membrane structure having excellent dispersibility in the aqueous phase and storage stability can be formed. The organic acid having a chelating effect is, for example, preferably citric acid, ethylenediaminetetraacetic acid (EDTA), etidronic acid, or pentetic acid and more preferably ethylenediaminetetraacetic acid (EDTA).

As the component (E), either a synthetic product or a commercially available product may be used.

For the lower limit, the content (in terms of free acid) of the component (E) in the dispersion according to the present invention is preferably 0.00001 mass% or more, 0.0001 mass% or more, 0.001 mass% or more, 0.01 mass% or more, 0.02 mass% or more, or 0.04 mass% or more, relative to the dispersion (total mass: 100 mass%). For the upper limit, the content (in terms of free acid) of the component (E) is preferably 3.0 mass% or less, 2.5 mass% or less, 2.0 mass% or less, 1.5 mass% or less, or 1.0 mass% or less, relative to the dispersion (total mass: 100 mass%).

For the lower limit of the content (in terms of free acid) of the component (E) in the dispersion according to the present invention is preferably 0.1 parts by mass or more and more preferably 0.2 parts by mass or more, per 100 parts by mass of the component (A). For the upper limit, the content (in terms of free acid) of the component (E) is more preferably 20 parts by mass or less and more preferably 10 parts by mass or less, per 100 parts by mass of the component (A).

In view of enhancing the solubility of both the component (D) and the component (E) to the dispersion and controlling the pH of the dispersion in a predetermined range, the dispersion of the present invention preferably includes both the component (D) and the component (E). Such a dispersion may be obtained by adding the component (D) and the component (E) separately or by adding a salt of the component (D) and the component (E).

The salt of the component (D) and the component (E) are not particularly limited, and examples thereof include lysine hydrochloride; sodium salts of citric acid such as trisodium citrate; sodium salts of phosphoric acid such as disodium hydrogen phosphate; sodium benzoate; sodium salts of ethylenediaminetetraacetic acid such as trisodium ethylenediaminetetraacetate (EDTA-3Na); and sodium salts of diethylenetriaminepentaacetic acid such as pentasodium diethylenetriaminepentaacetate (pentasodium pentetate).

When the dispersion contains the component (D) and the component (E), the mass ratio of the component (D) to the component (E), (D)/(E), is 0.2 to 10. The lower limit of the range of the mass ratio (D)/(E) may be 0.3, 0.4, or 0.5. The upper limit of the range of the mass ratio (D)/(E) may be 10, 9, 8, 7, 6, or 5. The range of the mass ratio (D)/(E) is preferably, for example, 0.5 to 5.

For the lower limit, the total content of the component (D) and the component (E) in the dispersion of the present invention is preferably 0.05 mass% or more and more preferably 0.1 mass% or more. For the upper limit, the total amount of the component (D) and the component (E) is preferably 4.5 mass% or less and more preferably 2 mass% or less.

### [Component (F)]

The dispersion according to the present invention may further contain a lipophilic compound as a component (F), in addition to the component (A), component (B), and component (C-2). When the component (A) having a high acid value is used in combination with the component (B) and the component (F), a lipid membrane structure in which the component (A) and the component (F) are orientated can be easily formed, and a lipid membrane structure having a high storage stability can be formed. Furthermore, a finer lipid membrane structure can be formed by using the component (F) in combination with the component (D) and/or the component (E). The component (F) may consist of one compound or two or more compounds.

Examples of the component (F) include sterols such as phytosterols, cholesterol, di(phytosteryl/octyldodecyl) lauroyl glutamate, phytosteryl oleate, and phytosteryl glucoside; triterpenes such as γ-oryzanol, glycyrrhizic acid, ursolic acid, and Centella asiatica extract (a mixture of asiatic acid, madecassic acid, and asiaticoside); lipophilic vitamins such as retinol, hydrogenated retinol, cholecalciferol, tocopherol, and ascorbic acid ester; carotenoids such as astaxanthin and β-carotene; coenzymes such as ubiquinone; hydrocarbons such as limonene, Vaseline, and squalane; ceramides such as ceramide EOS, ceramide NG (ceramide 2), ceramide NP (ceramide 3), ceramide AP (ceramide 6II), ceramide EOP (ceramide 1), dihydroxylignoceroyl phytosphingosine, cerebroside, glycosphingolipid, cetyl PG hydroxyethyl palmitamide; and polyphenols such as tetrahydrodiferuloylmethane and pterostilbene. In particular, a lipid membrane structure in which the component (A) and the component (F) are orientated is easily formed by combining the component (F), the component (A), and the component (B). In view of obtaining a dispersion having good storage stability and in view of enhancing the storage stability of a lipid membrane structure-containing composition including the dispersion, the component (F) is preferably at least one selected from the group consisting of phytosterols, cholesterol, γ-oryzanol, glycyrrhizic acid, ursolic acid, Centella asiatica extract (a mixture of asiatic acid, madecassic acid, and asiaticoside), hydrogenated retinol, tocopherol, astaxanthin, ubiquinone, ceramide NG, ceramide NP, ceramide AP, ceramide EOP, tetrahydrodiferuloylmethane, and pterostilbene.

As the component (F), either a synthetic product or a commercially available product may be used. Examples of the commercially available product include Phytosterol-SKP produced by Tama Biochemical Co., Ltd., TECA produced by Bayer, dl-α-Tocopherol produced by DSM Japan K.K., NIKKOL (registered trademark) Retinol H10, NIKKOL (registered trademark) VC-IP, and Squalane produced by Nikko Chemicals Co., Ltd., Astaxanthin-20C and γ-oryzanol produced by Oryza Oil & Fat Chemical Co., Ltd., Kaneka-Coenzyme Q10 produced by KANEKA CORPORATION, CERAMIDE 2 produced by Croda Japan K.K., and Ursolic Acid 90%, SabiWhite, and pTeroWhite produced by Sabinsa Japan Corporation.

For the lower limit, the content of the component (F) in the dispersion according to the present invention is, for example, 0.0000005 mass% or more, 0.000001 mass% or more, 0.00001 mass% or more, 0.0001 mass% or more, 0.001 mass% or more, 0.01 mass% or more, or 0.1 mass% or more, relative to the dispersion (total mass: 100 mass%). For the upper limit, the content of the component (F) is 9 mass% or less, 8 mass% or less, 7 mass% or less, 6 mass% or less, 5 mass% or less, 4 mass% or less, 3 mass% or less, 2 mass% or less, or 1 mass% or less, relative to the dispersion (total mass: 100 mass%).

For the lower limit, the content of the component (F) in the dispersion of the present invention is preferably 0.005 parts by mass or more, more preferably 0.05 parts by mass or more, further preferably 0.5 parts by mass or more, and particularly preferably 5 parts by mass or more, per 100 parts by mass of the component (A). For the upper limit, the content of the component (F) is preferably 60 parts by mass or less, more preferably 50 parts by mass or less, further preferably 40 parts by mass or less, and particularly preferably 30 parts by mass or less.

### [Other component]

The dispersion according to the present invention may further contain a component other than the above-mentioned components (A) to (F) (other component). The other component is not particularly limited, and examples thereof include a polyol compound other than the component (B), an oil agent, a surfactant, a moisturizing agent, a whitening agent, a coloring material, an alcohol, an amino acid, a sugar, a vitamin, a viscosity modifier, a polymer, a coloring agent, a powder, an ultraviolet ray absorber, a preservative, an antimicrobial agent, an antioxidant, a perfume, a cosmetic ingredient, an electrolyte, a fiber, and plant extract. These components may be used singly or in combination of two or more thereof. A lipid membrane structure encapsulating a cosmetic ingredient can be formed by incorporating, for example, a cosmetic ingredient, such as a moisturizing agent and a whitening agent, in the dispersion. For forming a lipid membrane structure encapsulating a cosmetic ingredient, the timing of incorporating the cosmetic ingredient is not particularly limited. The following is believed: until the concentration of the component (A) reaches the concentration determined depending on a target particle diameter, the particle diameter and structure (monolayer or multilayer) of a lipid membrane structure in a dispersion are changing, or in other words, the lipid membrane structure is in a reconstructable state; and on this occasion, the cosmetic ingredient existing in the dispersion is incorporated and encapsulated in the lipid membrane structure to be reconstructed. Based on this, a cosmetic ingredient for enhancing the feel of permeation and a cosmetic ingredient for reducing the feel of permeation and enhancing sustained release can be encapsulated in respective optimum lipid membrane structures by designing appropriately the concentration of the component (A) in the dispersion in terms of the particle diameter and structure (monolayer or multilayer) of the lipid membrane structure. Thus, a wide variety of lipid membrane structures for various cosmetic ingredients can be easily produced wherein optimum feel of permeation, sustained release, and so on have been designed for each lipid membrane structure, and by combining them, different types of highly functional products that satisfy various consumer needs can be conveniently produced.

### [Dispersing method in dispersing step]

As long as a lipid membrane structure of 10 nm or more and 200 nm or less is spontaneously formed in a dispersion in the dispersing step, the way to mix the component (A), component (B), and component (C-2) is not particularly limited. For example, in the dispersing step, the components may be mixed at once or in sequence. When the components are mixed in sequence, the order thereof is not particularly limited. For example, the component (A) and the component (B) are mixed, and the component (C-2) may be then added thereto and mixed therewith. Alternatively, the component (A) and the component (C-2) are mixed, and the component (B) may be then added thereto and mixed therewith. Alternatively, to the component (A), the component (B) and the component (C) may be simultaneously added (for example, a mixed solvent composed of the component (B) and the component (C-2) is added), followed by mixing. For example, when a dispersion is produced by using a component (D) and/or a component (E), in addition to the component (A), component (B), and component (C-2), the component (D) and/or the component (E) may be separately added, or may be mixed in advance and then added as a mixture. For example, when a dispersion is produced by using a component (D) and/or a component (E), in addition to the component (A), component (B), component (C-2), and component (F), the timing of addition of the component (F) is not particularly limited. For example, the component (A), component (B), component (C-2), and component (F) are mixed, the component (C-2), the component (D), and/or the component (E) may be then simultaneously or sequentially added thereto. Alternatively, in addition to the component (A), component (B), and component (C-2), the component (D) and/or the component (E) may be simultaneously or sequentially mixed therewith, and finally the component (F) may be then added thereto. When a dispersion is produced by further using a component described in [Additional component], the timing of addition of the component described in [Additional component] is not particularly limited, and may be appropriately selected depending on, for example, the solubility. For example, when the component is lipophilic, it may be added together with the component (F) or may be added last. When the component is water-soluble, it may be added together with the component (D) and/or the component (E) or may be added last.

In the dispersing step, the component (A), the component (B), and the component (C-2) are preferably mixed at the phase transition temperature of the component (A) or higher. In this case, the efficiency of forming a lipid membrane structure is more improved, and a dispersion having further excellent dispersion stability is obtained. For the upper limit, the mixing temperature in the dispersing step is, for example, 120°C or less, 110°C or less, 100°C or less, 95°C or less, or 90°C or less. For the lower limit, the mixing temperature is 40°C or more, 45°C or more, 50°C or more, 55°C or more, 60°C or more, 65°C or more, 70°C or more, 75°C or more, 80°C or more, or 85°C or more. The mixing temperature in the dispersing step is, for example, 40°C to 120°C, preferably 40°C to 100°C, and more preferably 60°C to 90°C. When a component (D), a component (E), and/or another cosmetic ingredient, for example, are incorporated as water-soluble components, these water-soluble components may be uniformly dissolved in the component (C-2) in advance and then incorporated. The temperature of dissolving the water-soluble components at that time is not limited to the above-mentioned mixing temperature, and the water-soluble components may be heated and dissolved at a temperature suitable for dissolving them. In a case in which the water-soluble components are thus uniformly dissolved in the component (C-2) and are then incorporated, they may be incorporated at any timing and temperature. For example, in a case in which all components are mixed in one container, water-soluble components may be heated and dissolved in the component (C-2) first, and the component (A) and the component (B) may be then added thereto and mixed therewith. Alternatively, water-soluble components may be heated and dissolved in the component (C-2) in another container once, and the resultant may be mixed with the component (A) and the component (B) at any timing and any temperature. The mixing time is also not particularly limited and is preferably 10 to 180 minutes.

Since the dispersion obtained by mixing the component (A), the component (B), and the component (C-2) forms spontaneously a fine lipid membrane structure even if there is no substantial mechanical shearing force, and the stirring may be performed at a stirring power that can uniformly mix these components. Specifically, a mixture solution obtained by mixing the component (A) and the component (B) may be added to the component (C-2) that is not being stirred, followed by mixing these components with a stirring power that can uniformly mix these components, or may be added while stirring the component (C-2). On this occasion, the stirring conditions are not particularly limited, and the stirring is performed, for example, using a known stirring means at a rotation frequency of 10 to 300 rpm. The mixture solution of the component (A) and the component (B) may be added at once or added in portions, or may be added in sequence at any addition rate using a known dropping means. The stirring method is not particularly limited, and since a high mechanical shearing force is not necessary, a known stirring means such as a magnetic stirrer (for example, a hot stirrer), a puddle mixer, a propeller mixer, or a planetary mixer can be used.

In an embodiment of the present invention, the dispersing step can be heating a mixture solution obtained by mixing a component (A) and a component (B) to the phase transition temperature of the component (A) or higher (for example, 80°C) (provided that the mixture solution of this case is in a state in which it does not correspond to a lipid membrane source agent) and mixing the resultant with a component (C-2) heated to the phase transition temperature of the component (A) or higher (for example, 80°C) to thereby obtain a dispersion. In another embodiment, the dispersing step can be mixing a component (A), a component (B), and a component (C-2) while heating them to the phase transition temperature of the component (A) or higher (for example, 80°C) to thereby obtain a dispersion.

Here, the particle diameter of the lipid membrane structure in the dispersion obtained by the dispersing step is the same as in the above-described lipid membrane structure-containing composition. This is because that in the present invention, the particle diameter of the lipid membrane structure included in a dispersion depends on the dispersing step.

In view of obtaining a finer lipid membrane structure, the pH of the dispersion is preferably 4.0 or more, more preferably 5.0 or more, further preferably 7.0 or more, and further more preferably 8.0 or more. In view of obtaining a lipid membrane structure with a uniform particle diameter (that is, a highly uniform dispersion), the pH of the dispersion is preferably 10.0 or less, more preferably 9.5 or less, and further more preferably 9.0 or less. Accordingly, the dispersion according to a preferable embodiment of the present invention has a pH of 4.0 to 10.0. Here, the "pH of a dispersion" is a pH of a dispersion measured at 25°C using a pH meter (HM-25R, produced by DKK-TOA CORPORATION) by a glass electrode method.

### [Mixing step]

In a method for producing a lipid membrane structure-containing composition of the present invention, the mixing step is performed as needed before the dispersing step. Namely, in an embodiment of the method for producing a lipid membrane structure-containing composition of the present invention, the mixing step is included. The mixing step is heating and uniformly mixing a component (A) and a component (B) to thereby obtain a lipid membrane source agent, before the dispersing step. That is, the mixing step is previously heating and uniformly mixing components for forming the lipid membrane structure to make a lipid membrane source agent in a mixed state in which the components are uniformly mixed. Here, the mixing in the dispersing step and the mixing step can be distinguished from each other by whether a mixture obtained is in a uniformly mixed state at the time when the component (C-2) is not included. For example, a lipid membrane source agent from the mixing step is a uniform and transparent liquid when heated and dissolved at the phase transition temperature of the component (A) or higher (for example, 80°C or higher), and even after it is left for a while or cooled, the uniform state is maintained with no deposition, separation, or precipitation of a part of the contained components. For example, even when the component (A) and the component (B) are heated to the phase transition temperature of the component (A) or higher (for example, 80°C) in the dispersing step, some components remain undissolved or the mixture is in a nonuniform and cloudy state, and a part of the components contained deposits, separates, or precipitates after the mixture is left for a while or cooled. Such a difference can distinguish the mixing in the dispersing step and the mixing step.

Accordingly, the lipid membrane source agent obtained by the mixing step becomes a uniform and transparent solution at the phase transition temperature of the component (A) or higher. When a solution including the component (A) and the component (B) or a solution including the component (A), the component (B), and the component (C-1) (i.e., component (C)) has undergone a process in which it was a transparent and uniform solution, the solution is a lipid membrane source agent, and the step of obtaining the solution is regarded as "mixing step".

In an embodiment including the mixing step, the dispersing step is mixing a lipid membrane source agent obtained in the mixing step and a component (C-2) to obtain a dispersion in which a lipid membrane structure of a particle diameter of 10 nm or more and 200 nm or less is spontaneously formed. That is, the dispersing step in this case is mixing a lipid membrane source agent including a component (A) and a component (B) with a component (C-2) to obtain a dispersion in which a lipid membrane structure of a particle diameter of 10 nm or more and 200 nm or less is spontaneously formed.

The lipid membrane source agent obtained in the mixing step may include the component (C-1), components (D) to (F), and a component described in the section [Additional component] as needed, in addition to the component (A) and the component (B). The preferable content of each component in the lipid membrane source agent will be described later, but preferable modes other than the content of each component are as described above. In an embodiment, the mixing step is mixing the component (A), the component (B), and optionally one or more selected from the group consisting of the component (C-1), the component (D), the component (E), and the component (F), to obtain a lipid membrane source agent.

The content of the component (A) in the lipid membrane source agent according to the present invention is not particularly limited. For example, for the lower limit, the content is 5 mass% or more, 6 mass% or more, 7 mass% or more, 8 mass% or more, 9 mass% or more, 10 mass% or more, 11 mass% or more, 12 mass% or more, 13 mass% or more, 14 mass% or more, 15 mass% or more, 16 mass% or more, 17 mass% or more, 18 mass% or more, or 19 mass% or more, relative to the lipid membrane source agent (total mass: 100 mass%). For the upper limit, the content of the component (A) in the lipid membrane source agent is 40 mass% or less, 35 mass% or less, 30 mass% or less, 25 mass% or less, or 20 mass% or less, relative to the lipid membrane source agent (total mass: 100 mass%). The content of the component (A) in the lipid membrane source agent is preferably 5 to 40 mass% and more preferably 10 to 20 mass%.

The content of the component (B) in the lipid membrane source agent according to the present invention is not particularly limited. For example, for the lower limit, the content is 15 mass% or more, 20 mass% or more, 25 mass% or more, 30 mass% or more, 35 mass% or more, 40 mass% or more, 45 mass% or more, or 50 mass% or more, relative to the lipid membrane source agent (total mass: 100 mass%). For the upper limit, the content of the component (B) in the lipid membrane source agent is 95 mass% or less, 90 mass% or less, 85 mass% or less, 80 mass% or less, 75 mass% or less, 70 mass% or less, 65 mass% or less, 60 mass% or less, or 55 mass% or less, relative to the lipid membrane source agent (total mass: 100 mass%). The content of the component (B) in the lipid membrane source agent is preferably 30 to 90 mass%, more preferably 40 to 80 mass%, and further more preferably 50 to 70 mass%.

In the lipid membrane source agent according to the present invention, the content of the component (B) exceeds 100 parts by mass per 100 parts by mass of the component (A). If the content of the component (B) is 100 parts by mass or less per 100 parts by mass of the component (A), a fine lipid membrane structure cannot be formed. Furthermore, the content of the component (B) is 110 parts by mass or more, 120 parts by mass or more, 130 parts by mass or more, 140 parts by mass or more, or 150 parts by mass or more, per 100 parts by mass of the component (A). When the content is 150 parts by mass or more, the component (A) is easily mixed more homogeneously in the lipid membrane source agent. A lipid membrane structure with excellent dispersibility in an aqueous phase can be formed by using the lipid membrane source agent. Furthermore, in view of obtaining a finer lipid membrane structure, the content of the component (B) is, for example, 160 parts by mass or more, 170 parts by mass or more, 180 parts by mass or more, 190 parts by mass or more, 200 parts by mass or more, 210 parts by mass or more, 220 parts by mass or more, 230 parts by mass or more, 240 parts by mass or more, 250 parts by mass or more, 260 parts by mass or more, 270 parts by mass or more, 280 parts by mass or more, 290 parts by mass or more, 300 parts by mass or more, 310 parts by mass or more, 320 parts by mass or more, 330 parts by mass or more, 340 parts by mass or more, 350 parts by mass or more, 360 parts by mass or more, 370 parts by mass or more, 380 parts by mass or more, 390 parts by mass or more, or 400 parts by mass or more, per 100 parts by mass of the component (A). For the upper limit, the content of the compound (B) per 100 parts by mass of the component (A) is not particularly limited, and even if it exceeds 2000 parts by mass per 100 parts by mass of the component (A), the solubility or dispersibility of the component (A) in the lipid membrane source agent is not affected, and it is simply uneconomical. Consequently, the content of the component (B) is, for example, 2000 parts by mass or less, 1900 parts by mass or less, 1800 parts by mass or less, 1700 parts by mass or less, 1600 parts by mass or less, 1500 parts by mass or less, 1400 parts by mass or less, 1300 parts by mass or less, 1200 parts by mass or less, 1100 parts by mass or less, 1000 parts by mass or less, 900 parts by mass or less, 800 parts by mass or less, 700 parts by mass or less, 600 parts by mass or less, or 500 parts by mass or less, per 100 parts by mass of the component (A). Accordingly, according to a preferable embodiment of the present invention, the content of the component (B) is preferably 110 to 2000 parts by mass per 100 parts by mass of the component (A).

In the lipid membrane source agent according to the present invention, the component (C-1) is added as needed, and the lower limit of its content is not particularly limited. For the lower limit, the content of the component (C-1) in the lipid membrane source agent is, for example, 0.5 mass% or more, 1 mass% or more, 2 mass% or more, 3 mass% or more, 4 mass% or more, 5 mass% or more, 6 mass% or more, 7 mass% or more, 8 mass% or more, 9 mass% or more, 10 mass% or more, 11 mass% or more, 12 mass% or more, 13 mass% or more, 14 mass% or more, 15 mass% or more, 16 mass% or more, 17 mass% or more, 18 mass% or more, 19 mass% or more, 20 mass% or more, 21 mass% or more, 22 mass% or more, 23 mass% or more, 24 mass% or more, or 25 mass% or more, relative to the lipid membrane source agent (total mass: 100 mass%). For the upper limit, the content of the component (C-1) in the lipid membrane source agent is 75 mass% or less, 70 mass% or less, 65 mass% or less, 60 mass% or less, 55 mass% or less, 50 mass% or less, 45 mass% or less, 40 mass% or less, 35 mass% or less, or 30 mass% or less, relative to the lipid membrane source agent (total mass: 100 mass%). The content of the component (C-1) in the lipid membrane source agent is preferably 0.5 to 70 mass%, more preferably 5 to 60 mass%, further preferably 15 to 50 mass%, and particularly preferably 20 to 40 mass%, relative to the lipid membrane source agent (total mass: 100 mass%).

For the lower limit, the mass ratio of the component (B) to the component (C-1) ("component (B) : component (C-1)") in the lipid membrane source agent according to the present invention is 25 : 75 or more, 30 : 70 or more, 35 : 65 or more, 40 : 60 or more, 45 : 55 or more, or 50 : 50 or more. For the lipid membrane source agent, the component (C-1) is added as needed, and thus the lower limit of its content is not particularly limited. Accordingly, the upper limit of the mass ratio of the component (B) to the component (C-1) ("component (B) : component (C-1)") is not particularly limited. For the upper limit, the mass ratio of the component (B) to the component (C-1) is, for example, 99 : 1 or less, 90 : 10 or less, or 80 : 20 or less. The mass ratio of the component (B) to the component (C-1) is preferably 25 : 75 or more and 99 : 1 or less, more preferably 30 : 70 or more and 90 : 10 or less, and further preferably 35 : 65 or more and 80 : 20 or less.

In the lipid membrane source agent according to the present invention, the component (D) is added as needed, and thus the lower limit of its content is not particularly limited. For the lower limit, the content of the component (D) in the lipid membrane source agent is, for example, 0.01 mass% or more or 0.02 mass% or more, relative to the lipid membrane source agent (total mass: 100 mass%), and for the upper limit, the content is 2 mass% or less, or 1 mass% or less, relative to the lipid membrane source agent (total mass: 100 mass%). The content of the component (D) in the lipid membrane source agent is preferably 0.01 to 2 mass% and more preferably 0.02 to 1 mass%.

In the lipid membrane source agent according to the present invention, the component (D) is added as needed, and thus the lower limit of its content is not particularly limited. For the lower limit, the content of the component (D) in the lipid membrane source agent is, for example 0.05 mass% or more or 0.1 mass% or more, per 100 mass% of the component (A), and for the upper limit, the content is 10 mass% or less, 9 mass% or less, 8 mass% or less, 7 mass% or less, 6 mass% or less, or 5 mass% or less, per 100 mass% of the component (A). In the lipid membrane source agent, the content of the component (D) is preferably 0.05 to 10 mass% and more preferably 0.1 to 5 mass%, per 100 mass% of the component (A).

In the lipid membrane source agent according to the present invention, the component (E) is added as needed, and the lower limit of its content is not particularly limited. For the lower limit, the content (in terms of free acid) of the component (E) in the lipid membrane source agent is, for example, 0.02 mass% or more or 0.04 mass% or more, and the upper limit is 4 mass% or less, 3 mass% or less, or 2 mass% or less, relative to the lipid membrane source agent (total mass: 100 mass%). In the lipid membrane source agent, the content (in terms of free acid) of the component (E) is preferably 0.02 to 4 mass% and more preferably 0.04 to 2 mass%, relative to the lipid membrane source agent (total mass: 100 mass%).

In the lipid membrane source agent according to the present invention, the component (E) is added as needed, and thus the lower limit of its content is not particularly limited. For the lower limit, the content (in terms of free acid) of the component (E) in the lipid membrane source agent is, for example, 0.1 parts by mass or more or 0.2 parts by mass or more, and the upper limit is 20 parts by mass or less, 19 parts by mass or less, 18 parts by mass or less, 17 parts by mass or less, 16 parts by mass or less, 15 parts by mass or less, 14 parts by mass or less, 13 parts by mass or less, 12 parts by mass or less, 11 parts by mass or less, or 10 parts by mass or less, per 100 parts by mass of the component (A). In the lipid membrane source agent, the content (in terms of free acid) of the component (E) is preferably 0.1 to 20 parts by mass and more preferably 0.2 to 10 parts by mass, per 100 parts by mass of the component (A).

When the lipid membrane source agent according to the present invention contains the component (D) and the component (E), the mass ratio of the component (D) to the component (E), (D)/(E), is, for example, 0.2 to 10. The lower limit of the range of the mass ratio (D)/(E) is, for example, 0.2, 0.3, 0.4, or 0.5, and the upper limit is 10, 9, 8, 7, 6, or 5. The range of the mass ratio (D)/(E) is preferably 0.5 to 5, for example.

When the lipid membrane source agent according to the present invention contains the component (D) and the component (E), the total content of the component (D) and the component (E) is, for example, 0.05 mass% or more or 0.1 mass% or more, relative to the lipid membrane source agent (total mass: 100 mass%), for the lower limit. For the upper limit, the total content is 4.5 mass% or less, 4.0 mass% or less, 3.5 mass% or less, 3.0 mass% or less, 2.5 mass% or less, or 2 mass% or less, relative to the lipid membrane source agent (total mass: 100 mass%), for the lower limit. In the lipid membrane source agent, the total content of the component (D) and the component (E) is preferably 0.05 to 4 mass% and more preferably 0.1 to 2 mass%, relative to the lipid membrane source agent (total mass: 100 mass%).

In the lipid membrane source agent according to the present invention, the component (F) is added as needed, and the lower limit of its content is not particularly limited. For the lower limit, the content of the component (F) in the lipid membrane source agent is, for example, 0.00025 mass% or more, 0.001 mass% or more, 0.01 mass% or more, 0.1 mass% or more, or 1 mass% or more, relative to the lipid membrane source agent (total mass: 100 mass%). For the upper limit, the content is 12 mass% or less, 11 mass% or less, 10 mass% or less, 9 mass% or less, 8 mass% or less, 7 mass% or less, or 6 mass% or less, relative to the lipid membrane source agent (total mass: 100 mass%). The content of the component (F) in the lipid membrane source agent is preferably 0.001 to 12 mass%, more preferably 0.01 to 10 mass%, further more preferably 0.1 to 8 mass%, and particularly preferably 1 to 6 mass%, relative to the lipid membrane source agent (total mass: 100 mass%).

In the lipid membrane source agent according to the present invention, the component (F) is added as needed, and thus the lower limit of the content is not particularly limited. For the lower limit, the content of the component (F) in the lipid membrane source agent is, for example, 0.005 parts by mass or more, 0.05 parts by mass or more, 0.5 parts by mass or more, or 5 parts by mass or more, per 100 parts by mass of the component (A). For the upper limit, the content is 60 parts by mass or less, 50 parts by mass or less, 40 parts by mass or less, or 30 parts by mass or less, per 100 parts by mass of the component (A). The content of the component (F) in the lipid membrane source agent is preferably 0.005 to 60 parts by mass, more preferably 0.05 to 50 parts by mass, further more preferably 0.5 to 40 parts by mass, and particularly preferably 5 to 30 parts by mass, per 100 parts by mass of the component (A).

In the mixing step, the components may be mixed at once or mixed in sequence. When the components are mixed in sequence, the order thereof is not particularly limited. For example, the component (A) and the component (B) may be mixed, and the component (C-1) may be then added thereto and mixed therewith; the component (A) and the component (C-1) may be mixed, and the component (B) may be then added thereto and mixed therewith; or the component (B) and the component (C-1) may be simultaneously added (for example, a mixture of the component (B) and the component (C-1) is added) to and mixed with the component (A). For example, when a lipid membrane source agent is produced by using the component (D) and/or the component (E) in addition to the component (A), the component (B), and the component (C-1), the component (D) and/or the component (E) may be added separately, or they may be mixed with the component (C-1) in advance, followed by adding the mixture to a mixture of the component (A) and the component (B). For example, when a lipid membrane source agent is produced by using the component (D) and/or the component (E) in addition to the component (A), the component (B), the component (C-1), and the component (F), the timing of addition of the component (F) is not particularly limited. For example, the component (A), the component (B), and the component (F) may be mixed, and the component (C-1), the component (D), and/or the component (E) may be added thereto simultaneously or sequentially. Alternatively, in addition to the component (A) and the component (B), the component (C-1), the component (D), and/or the component (E) may be mixed simultaneously or sequentially, and the component (F) may be added last. When a lipid membrane source agent is produced by further using a component described in [Additional component], the timing of addition of the component described in [Additional component] is not particularly limited, and can be appropriately selected depending on, for example, the solubility. For example, if the component is lipophilic, it may be added together with the component (F) or may be added last. Alternatively, if the component is water-soluble, it may be added together with the component (C-1), the component (D), and/or the component (E) or may be added last.

In the mixing step, the mixing temperature is not particularly limited, but the component (A) and the component (B) are preferably mixed at the phase transition temperature of the component (A) or higher. In this case, the miscibility of the component (A) to the component (B) is improved, and a lipid membrane source agent having excellent dispersibility in the component (C-2) as the aqueous phase can be obtained. For the upper limit, the mixing temperature is, for example, 120°C or less, 110°C or less, 100°C or less, 95°C or less, or 90°C or less. For the lower limit, the mixing temperature is, for example, 40°C or more, 45°C or more, 50°C or more, 55°C or more, 60°C or more, 65°C or more, 70°C or more, 75°C or more, 80°C or more, or 85°C or more. The mixing temperature is preferably 25°C to 120°C, more preferably 40°C to 100°C, and further preferably 60°C to 90°C. The mixing time is also not particularly limited, but is preferably 10 to 180 minutes. The mixing method is not particularly limited, and since it does not require a high mechanical shearing force, a known mixing means such as a magnetic stirrer (for example, a hot stirrer), a puddle mixer, a propeller mixer, or a planetary mixer can be used.

### [Concentration-adjusting step]

The method for producing a lipid membrane structure-containing composition of the present invention may include, in addition to the dispersing step, the step of further mixing the dispersion and a component (C-3) to adjust the concentration (hereinafter, "concentration-adjusting step"). When the producing method of the present invention includes the concentration-adjusting step, a lipid membrane structure-containing composition is obtained through the concentration-adjusting step.

In the method for producing a lipid membrane structure-containing composition of the present invention, the concentration-adjusting step is performed as needed after the dispersing step. That is, in an embodiment, the method for producing a lipid membrane structure-containing composition of the present invention includes the concentration-adjusting step. The concentration-adjusting step is, after the dispersing step, further adding a component (C-3) to the dispersion obtained in the dispersing step such that the concentration of the component (A) included in the lipid membrane structure-containing composition is the target concentration. That is, the concentration-adjusting step is obtaining a lipid membrane structure-containing composition by mixing the dispersion obtained in the dispersing step and the component (C-3) in an amount necessary for adjusting the concentration of the component (A) included in the lipid membrane structure-containing composition to a target concentration.

In a case in which the mixing step is not carried out, the amount of the component (C-3) mixed with the dispersion in the concentration-adjusting step is the amount obtained by subtracting the amount of the component (C-2) used in the dispersing step from the amount of the component (C) to be included in the target lipid membrane structure-containing composition. In a case in which the mixing step is carried out, the amount of the component (C-3) is the amount obtained by subtracting the amount of the component (C-1) used in the mixing step and the amount of the component (C-2) used in the dispersing step from the amount of the component (C) to be included the target lipid membrane structure-containing composition. Alternatively, the amount of the component (C-3) for dilution may be calculated such that the concentration of the component (A) in the dispersion becomes equal to the concentration of the component (A) in the target lipid membrane structure-containing composition.

The method for mixing the dispersion and the component (C-3) in the concentration-adjusting step is not particularly limited, and it may be a method including adding the component (C-3) to the dispersion or a method including adding the dispersion to the component (C-3). In view of improving the dispersibility of the lipid membrane structure, the latter method is preferable. Accordingly, the method for producing a lipid membrane structure-containing composition according to an embodiment of the present invention includes adding a component (C-3) to the dispersion.

The upper limits of the contents (concentrations) of the components (A) to (C) in the lipid membrane structure-containing composition are omitted here, because they are the same as of the contents of the respective components in the dispersion described above, considering a case in which the concentration-adjusting step is not performed. The lower limits of the components (A) and (B) are not particularly limited because the dispersion is diluted in the concentration-adjusting step; however, the lower limits can be calculated as values when diluted according to the concentration-adjusting step, on the basis of the contents of the respective components in the dispersion described above.

Here, the particle diameter of the lipid membrane structure in the lipid membrane structure-containing composition obtained in the concentration-adjusting step is the same as the particle diameter of the lipid membrane structure in the dispersion. This is because, in the present invention, the particle diameter of the lipid membrane structure included in the dispersion depends on the dispersing step, and even if dilution is then performed by the concentration-adjusting step, the particle diameter can be maintained.

In the concentration-adjusting step, the mixing temperature is not particularly limited, and the concentration-adjusting step may be performed at the phase transition temperature of the component (A) or higher or at a temperature lower than the phase transition temperature of the component (A). In the former, for the lower limit, the mixing temperature is, for example, 40°C or more, 45°C or more, 50°C or more, 55°C or more, 60°C or more, or 65°C or more, and for the upper limit, the mixing temperature is, for example, 100°C or less, 95°C or less, or 90°C or less. In the latter, for the upper limit, the mixing temperature, is for example, less than 40°C, 35°C or less, 30°C or less, 25°C or less, 20°C or less, 15°C or less, or 10°C or less, and for the lower limit, the mixing temperature, is, for example, -10°C or more, -5°C or more, 0°C or more, or 5°C or more. The mixing time is not particularly limited, and is, for example, 10 to 60 minutes.

Mixing of the dispersion and the component (C-3) may be performed by addition (of the dispersion to the component (C-3)) without stirring the aqueous phase or while stirring the component (C-3), or may be performed by adding the component (C-3) to the dispersion. On this occasion, the stirring conditions of the component (C-3) and/or the dispersion are not particularly limited, and, for example, the stirring is performed using a known stirring means at a rotation frequency of 10 to 300 rpm. The dispersion and/or the component (C-3) may be added at once or added in portions or may be added in sequence at any addition rate using a known dropping means.

In the concentration-adjusting step, the temperatures of the dispersion and the component (C-3) are not particularly limited and may be the phase transition temperature of the component (A) or higher or a temperature lower than the phase transition temperature of the component (A). In the former, the temperature is, for example, 40°C or more, 45°C or more, 50°C or more, 55°C or more, 60°C or more, or 65°C or more, and the upper limit is 100°C or less, 95°C or less, or 90°C or less, for the lower limit. In the latter, the temperature is, for example, less than 40°C, 35°C or less, 30°C or less, 25°C or less, 20°C or less, 15°C or less, or 10°C or less, and the lower limit is -10°C or more, -5°C or more, 0°C or more, or 5°C or more, for the upper limit. The temperatures of the both may be the same or different. For example, the temperature of one of the dispersion and the component (C-3) may be the phase transition temperature of the component (A) or higher, and the temperature of the other of the component (C-3) and the dispersion may be less than the phase transition temperature of the component (A).

Here, in the concentration-adjusting step, the component (D), the component (E), and/or the component (F), in addition to the component (C-3), may be mixed with the dispersion. In this case, the component (D), the component (E), and/or the component (F) may be added separately, or may be mixed in advance and added as a mixture. When a lipid membrane structure-containing composition is produced by further using a component described in [Additional component], the timing of addition of the component described in [Additional component] is not particularly limited, and can be appropriately selected depending on, for example, the solubility. For example, if the component is lipophilic, it may be added together with the component (F) or may be added last. Alternatively, if the component is water-soluble, it may be added together with the component (D) and/or the component (E) or may be added last. The upper limits of the contents of these components (D) to (F) and the component described in the section [Additional component] are the same as of the contents of the respective components in the dispersion described above, considering a case in which the concentration-adjusting step is not performed. The lower limits are not particularly limited because the dispersion is diluted in the concentration-adjusting step; however, the lower limits can be calculated as values when diluted according to the concentration-adjusting step, on the basis of the contents of the respective components in the dispersion.

Here, in the concentration-adjusting step, another component may be further added, as long as the lipid membrane structure can be formed. Examples of such a component include a polyol compound other than the component (B), an oil agent, a surfactant, a moisturizing agent, a whitening agent, a coloring material, an alcohol, an amino acid, a sugar, a vitamin, a viscosity modifier, a polymer, a coloring agent, a powder, an ultraviolet ray absorber, a preservative, an antimicrobial agent, an antioxidant, a perfume, a cosmetic ingredient, an electrolyte, a fiber, and plant extract. The timing of addition of these additional components is also not particularly limited and can be appropriately selected depending on, for example, the solubility. Even if an additional component is added in addition to the component (C-3) in the concentration-adjusting step as described above, the particle diameter of the lipid membrane structure is not affected by the addition of the additional component in the concentration-adjusting step, since the particle diameter of the obtained lipid membrane structure depends on the concentration of the component (A) in the dispersion obtained in the dispersing step.

In method for producing a lipid membrane structure-containing composition according to the present invention, other steps such as purification (e.g., filtration), cooling, and storage may be further performed, in addition to the dispersing step, mixing step, and concentration-adjusting step.

### <Application of lipid membrane structure-containing composition>

The lipid membrane structure-containing composition according to the present invention can be used directly, or the lipid membrane structure-containing composition according to the present invention may be further dispersed in a dispersion medium and used. The dispersion medium in this case is not particularly limited, and examples thereof include water, a polyol compound, and a mixture thereof. The polyol compound may include, for example, a polyol compound represented by a formula (1), and examples include 1,3-butylene glycol, dipropylene glycol, tripropylene glycol, glycerin, diglycerin, propylene glycol, and 1,2-pentanediol.

According to the lipid membrane structure-containing composition according to the present invention, a fine lipid membrane structure can be spontaneously formed even if there is no substantial mechanical shearing force, and at the same time, the fine lipid membrane structure can encapsulate a water-soluble ingredient or a lipophilic ingredient. Consequently, not only the producing cost at a producing plant can be reduced, but also it is possible to create a system for preparing, at time of use, a cosmetic or an external preparation for the skin containing a lipid membrane structure-containing composition encapsulating an arbitrary water-soluble or lipophilic cosmetic ingredient based on counselling at stores such as a cosmetic specialty store, and a wide range of use of the lipid membrane structure can be expected.

The lipid membrane structure-containing composition of the present invention is used as a cosmetic or an external preparation for the skin, as it is, or also can be incorporated in a cosmetic or an external preparation for the skin to impart skin-caring effects such as a moisturizing effect. Accordingly, an embodiment of the present invention is a cosmetic containing the lipid membrane structure-containing composition. An embodiment of the present invention is an external preparation for the skin containing the lipid membrane structure-containing composition. Accordingly, the present invention provides a method for producing a cosmetic, the method including producing a lipid membrane structure-containing composition by the producing method described above. The present invention also provides a method for producing an external preparation for the skin, the method including producing a lipid membrane structure-containing composition by the producing method described above.

The form of the cosmetic or the external preparation for the skin of the present invention is not particularly limited as long as the lipid membrane structure is stably blended, and examples thereof include lotion, gel, emulsion, cream, shampoo, and facial cleanser forms. In view of utilizing the transparent appearance, feel of permeation when applied, and high stability due to containing the fine lipid membrane structure (for example, of a particle diameter of 200 nm or less) of the present invention, a preferred example is incorporation into a low-viscosity lotion in which it is otherwise difficult to incorporate a lipid membrane structure.

The cosmetic or the external preparation for the skin of the present invention may further contain, in addition to the lipid membrane structure-containing composition, a component that is usually used in cosmetics or external preparations for the skin as long as that does not impair the effect of the present invention. Examples of such a component include, but not limited to, a polyol compound other than the component (B), an oil agent, a surfactant, a moisturizing agent, a whitening agent, a coloring material, an alcohol, an amino acid, a vitamin, a viscosity modifier, a polymer, a coloring agent, a powder, an ultraviolet ray absorber, a preservative, an antimicrobial agent, an antioxidant, a perfume, a cosmetic ingredient, an electrolyte, a pH adjuster, a fiber, water, and plant extract.

The concentration of the lipid membrane structure in the cosmetic or external preparation for the skin containing the lipid membrane structure-containing composition of the present invention is preferably 0.0001 mass% or more, more preferably 0.01 mass% or more, and further preferably 1 mass% or more, in view of obtaining a skin-caring effect of the lipid membrane structure. For the upper limit, the concentration is not particularly limited, and is, for example, less than 5 mass%.

Embodiments of the present invention have been described in detail, but it is apparent that these embodiments are illustrative and exemplary and are not restrictive, and the scope of the present invention should be interpreted by the appended claims.

The present invention encompasses the following modes and aspects.

[1] A method for producing a lipid membrane structure-containing composition including:
   (A) a hydrogenated phospholipid having an acid value of 5 mg KOH/g or more;
   (B) a compound represented by a following formula 1: 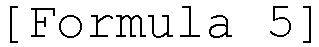 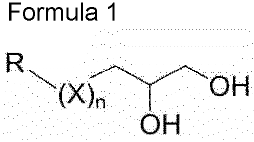 wherein
      R is a substituted or unsubstituted alkyl group having 2 to 6 carbon atoms or a substituted or unsubstituted cycloalkyl group having 3 to 6 carbon atoms,
      X is -O-, -C(=O)O-, or -O-C(=O)-, and
      n is 0 or 1; and
   (C) water;
   the method comprising:
   a dispersing step of mixing the component (A), the component (B), and the component (C) to obtain a dispersion in which a lipid membrane structure having a particle diameter of 10 nm or more and 200 nm or less is spontaneously formed, wherein
   the dispersing step includes determining a concentration of the component (A) in the dispersion depending on a target particle diameter of the lipid membrane structure included in the lipid membrane structure-containing composition.
[2] The method for producing a lipid membrane structure-containing composition according to the above [1], wherein a content of the component (B) is more than 100 parts by mass per 100 parts by mass of the component (A).
[3] The method for producing a lipid membrane structure-containing composition according to the above [1] or [2], wherein a concentration of the component (A) in the dispersion is 0.01 to 15 mass%.
[4] The method for producing a lipid membrane structure-containing composition according to any one of the above [1] to [3], further comprising a concentration-adjusting step of mixing the dispersion and the component (C) to obtain the lipid membrane structure-containing composition.
[5] The method for producing a lipid membrane structure-containing composition according to any one of the above [1] to [4], wherein the mixing in the dispersing step is performed at a phase transition temperature of the component (A) or higher.
[6] A method for producing a cosmetic, comprising producing a lipid membrane structure-containing composition by the producing method according to any one of the above [1] to [5].
[7] A method for producing an external preparation for the skin, comprising producing a lipid membrane structure-containing composition by the producing method according to any one the above [1] to [5].

### Examples

Effects of the present invention will be described by way of the following Examples and Comparative Examples. However, the technical scope of the present invention is not limited to the following Examples only. In the following Examples, the procedures were performed at room temperature (20°C to 25°C) unless otherwise specified. Unless otherwise specified, "%" and "part(s)" means "mass%" and "part(s) by mass", respectively.

### <Preparation of lipid membrane structure-containing composition>

The lipid membrane structure-containing composition was prepared by the following method. The hydrogenated lecithin was prepared by purchasing multiple commercially available soybean-derived hydrogenated lecithins having different acid values (acid value: 0.3 to 30.8 mg KOH/g) and mixing them at a ratio to give an intended acid value. The phase transition temperature of the hydrogenated lecithin of the component (A) or component (A') used in Examples and Comparative Examples were 40°C to 50°C.

### [Examples 1-0 and 1-1, Examples 2-0 and 2-1, Comparative Examples 1-0 and 1-1, and Comparative Examples 2-0 and 2-1]

To a 100-mL beaker, 0.1 parts by mass or 1 part by mass of a component (A): hydrogenated lecithin (acid value: 6.1 mg KOH/g, 13.9 mg KOH/g, or 17.3 mg KOH/g) or a component (A'): hydrogenated lecithin (acid value: 0.3 mg KOH/g) and 0.4 parts by mass or 4 parts by mass of a component (B): 1,2-pentanediol (in Tables 1 and 2, written as "pentanediol") or a component (B'): propylene glycol were added, followed by stirring using a hot stirrer at 80°C for 20 minutes to prepare a lipid membrane source agent. Then, to a 200-mL beaker, 99.5 mL or 95 mL of a component (C-2): purified water (in Tables 1 and 2, written as "water (for dispersion)") was added to provide an aqueous phase. The aqueous phase was heated to 80°C, and the above-prepared lipid membrane source agent at 80°C was added thereto while stirring at 100 rpm. After the addition was complete, stirring was performed at 80°C for 2 minutes to prepare a dispersion. The dispersion was used as the lipid membrane structure-containing composition in Examples 1-0 and 1-1, Examples 2-0 and 2-1, Comparative Examples 1-0 and 1-1, and Comparative Examples 2-0 and 2-1.

### [Examples 3-0 and 3-1]

To a 100-mL beaker, 0.1 parts by mass or 1 part by mass of a component (A): hydrogenated lecithin (acid value: 23.1 mg KOH/g), 0.4 parts by mass or 4 parts by mass of a component (B): 1,2-pentanediol, and 0.013 parts by mass or 0.13 parts by mass of a component (F): phytosterols were added, followed by stirring using a hot stirrer at 80°C for 20 minutes, and then 0.15 parts by mass or 1.49 parts by mass of a component (C-1): purified water heated to 80°C (in Table 1, written as "water (for source agent)"), 0.003 parts by mass or 0.03 parts by mass of a component (D): arginine, and 0.001 parts by mass or 0.01 parts by mass of a component (E): citric acid were added thereto, followed by stirring using a hot stirrer at 80°C for 5 minutes to prepare a lipid membrane source agent. Subsequently, to a 200-mL beaker, 99.33 mL or 93.34 mL of a component (C-2): purified water (in Table 1, written as "water (for dispersion)") was added to provide an aqueous phase. The aqueous phase was heated to 80°C, and the above-prepared lipid membrane source agent at 80°C was added thereto while stirring at 100 rpm. After the addition was complete, stirring was performed at 80°C for 2 minutes to prepare a dispersion. The dispersion was used as the lipid membrane structure-containing composition in Examples 3-0 and 3-1.

### [Comparative Examples 3-0 and 3-1]

To a 100-mL beaker, 0.1 parts by mass or 1 part by mass of a component (A): hydrogenated lecithin (acid value: 13.9 mg KOH/g), 0.45 parts by mass or 4.5 parts by mass of a component (B'): propylene glycol, and 0.45 parts by mass or 4.5 parts by mass of a component (B'): glycerin were added, followed by stirring using a hot stirrer at 80°C for 20 minutes to prepare a lipid membrane source agent. Then, to a 200-mL beaker, 99 mL or 90 mL of a component (C-2): purified water (in Table 2, written as "water (for dispersion)") was added to provide an aqueous phase. The aqueous phase was heated to 80°C, and the above-prepared lipid membrane source agent at 80°C was added thereto while stirring at 100 rpm. After the addition was complete, stirring was performed at 80°C for 2 minutes to prepare a dispersion. The dispersion was used as the lipid membrane structure-containing composition in Comparative Examples 3-0 and 3-1.

### [Examples 1-2 to 1-5, Examples 2-2 to 2-5, Comparative Examples 1-2 to 1-5, and Comparative Examples 2-2 to 2-5]

To a 100-mL beaker, 1 part by mass of a component (A): hydrogenated lecithin or a component (A'): hydrogenated lecithin having an acid value (unit: mg KOH/g) shown in Table 1 or 2 and 4 parts by mass of a component (B): 1,2-pentanediol or a component (B'): propylene glycol were added, followed by stirring using a hot stirrer at 80°C for 20 minutes to prepare a lipid membrane source agent. Subsequently, a component (C-2): purified water heated to 80°C (in an amount written in "water (for dispersion)" in Table 1 or 2) was added to the lipid membrane source agent, followed by stirring using a hot stirrer at 80°C for 5 minutes to prepare a dispersion. Then, to a 200-mL beaker, a component (C-3): purified water (in an amount written in "water (for adjustment of concentration)" in table 1 or 2) was added to provide an aqueous phase. The aqueous phase was heated to 80°C, and the above-prepared dispersion at 80°C was added thereto while stirring at 100 rpm. After the addition was complete, stirring was performed at 80°C for 2 minutes to prepare a lipid membrane structure-containing composition.

### [Examples 3-2 to 3-5]

To a 100-mL beaker, 1 part by mass of a component (A): hydrogenated lecithin having an acid value (unit: mg KOH/g) described in Table 1, 4 parts by mass of a component (B): 1,2-pentanediol, and 0.13 parts by mass of a component (F): phytosterols were added, followed by stirring using a hot stirrer at 80°C for 20 minutes, and then 1.49 parts by mass of a component (C-1): purified water heated to 80°C (in Table 1, written as "water (for source agent)"), 0.03 parts by mass of a component (D): arginine, and 0.01 parts by mass of a component (E): citric acid were added thereto, followed by stirring using a hot stirrer at 80°C for 5 minutes to prepare a lipid membrane source agent. Then, a component (C-2): purified water (in an amount written in "water (for dispersion)" in Table 1) heated to 80°C was added thereto, followed by stirring using a hot stirrer at 80°C for 5 minutes to prepare a dispersion. Subsequently, to a 200-mL beaker, a component (C-3): purified water (in an amount written in "water (for adjustment of concentration)" in Table 1) was added to provide an aqueous phase. The aqueous phase was heated to 80°C, and the above-prepared dispersion at 80°C was added thereto while stirring at 100 rpm. After the addition was complete, stirring was performed at 80°C for 2 minutes to prepare a lipid membrane structure-containing composition.

### [Comparative Examples 3-2 to 3-5]

To a 100-mL beaker, 1 part by mass of a component (A): hydrogenated lecithin (acid value: 13.9 mg KOH/g), 4.5 parts by mass of a component (B'): propylene glycol, and 4.5 parts by mass of a component (B'): glycerin were added, followed by stirring using a hot stirrer at 80°C for 20 minutes to prepare a lipid membrane source agent. Subsequently, to a 200-mL beaker, a component (C-2): purified water (in an amount written in "water (for dispersion)" in Table 2) was added to provide an aqueous phase. The aqueous phase was heated to 80°C, and the above-prepared lipid membrane source agent at 80°C was added thereto while stirring at 100 rpm. After the addition was complete, stirring was performed at 80°C for 2 minutes to prepare a dispersion. Then, to a 200-mL beaker, a component (C-3): purified water (in an amount written in "water (for adjustment of concentration)" in Table 2) was added to provide an aqueous phase. The aqueous phase was heated to 80°C, and the above-prepared dispersion at 80°C was added thereto while stirring at 100 rpm. After the addition was complete, stirring was performed at 80°C for 2 minutes to prepare a lipid membrane structure-containing composition.

In Tables 1 and 2, a blank indicates that the agent is not included.

### <Evaluation method>

### [Particle diameter/polydispersity index]

Regarding the above-prepared lipid membrane structure-containing compositions, the particle diameter (based on scattered light intensity) and polydispersity index (PDI) of the lipid membrane structure were measured by cumulant analysis using a dynamic light scattering measuring apparatus (produced by Malvern Instruments, Zetasizer Nano ZSP). The measurement results of the particle diameter and polydispersity index of the lipid membrane structure-containing compositions are shown in Tables 1 and 2. Regarding the lipid membrane structure-containing compositions obtained by the method including a concentration-adjusting step, the particle diameter of the lipid membrane structure in the dispersion obtained by the dispersing step was measured, and as a result, it was confirmed that the particle diameter and the polydispersity index of the lipid membrane structure in the dispersion were the same as the particle diameter and the polydispersity index of the lipid membrane structure in the lipid membrane structure-containing composition after the concentration-adjusting step.

### [Cryo-TEM observation]

Regarding the lipid membrane structure-containing compositions prepared in Examples 1-0 to 1-5, 2-0 to 2-5, and 3-0 to 3-5, Cryo-TEM observation was performed using a transmission electron microscope (produced by Hitachi High-Tech Corporation, H-7650). Regarding the lipid membrane structure-containing compositions prepared in Examples 3-1 and 3-3, the observation photographs obtained as a result of the Cryo-TEM observation are shown in (a) and (b) of Figure 1. In the observation photograph shown in Figure 1(a), a ring-like image shows a monolayer lamellar liposome structure, a bar-like image shows a bicelle structure, and formation of a monolayer lamellar lipid membrane structure was thus confirmed. In the observation photograph shown in Figure 1(b), a multilayer ring-like image shows a multilayer lamellar liposome structure, and formation of a multilayer lamellar lipid membrane structure was thus confirmed.

The particle diameter, polydispersity index, and Cryo-TEM observation results of each lipid membrane structure are shown in Tables 1 and 2 below. On the lipid membrane structure-containing compositions in Comparative Examples 1-0 to 1-5, it was not possible to measure the particle diameter due to poor dispersion. In Table 1, the results of Cryo-TEM observation are shown in the line "Membrane structure of lipid membrane structure" as "monolayer" when a monolayer lamellar structure was observed and as "multilayer" when a multilayer lamellar structure was observed. In the lipid membrane structure-containing compositions prepared in Comparative Examples, Cryo-TEM observation was not performed, since the particle diameter exceeded 200 nm.

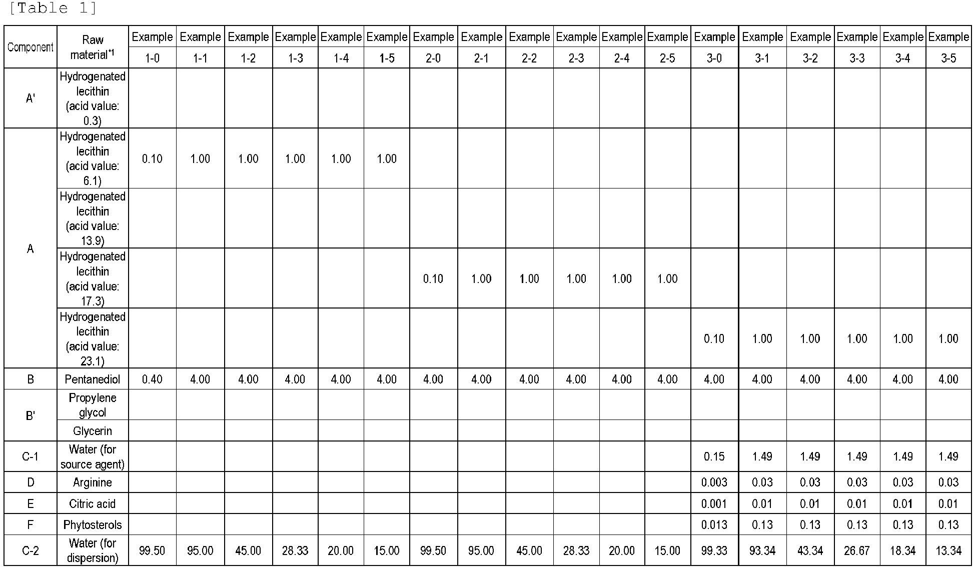

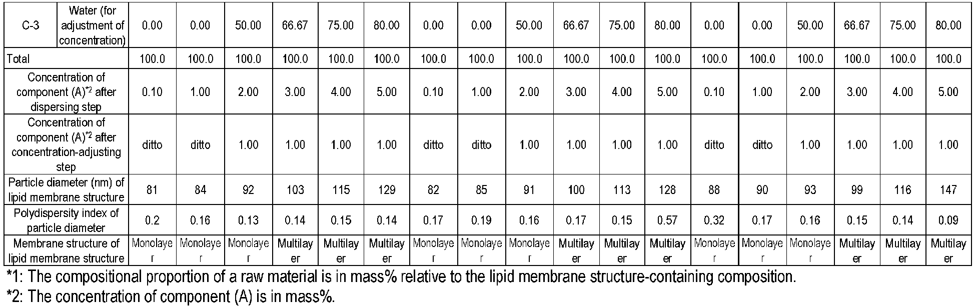

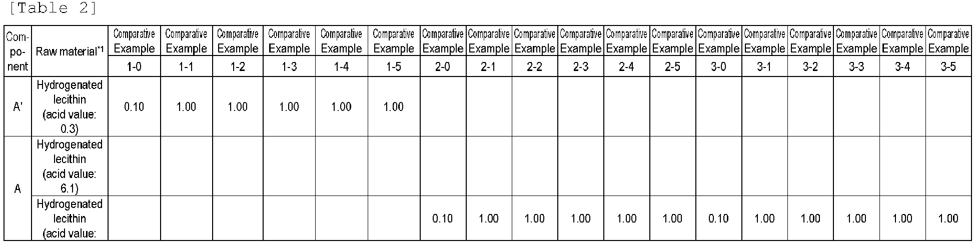

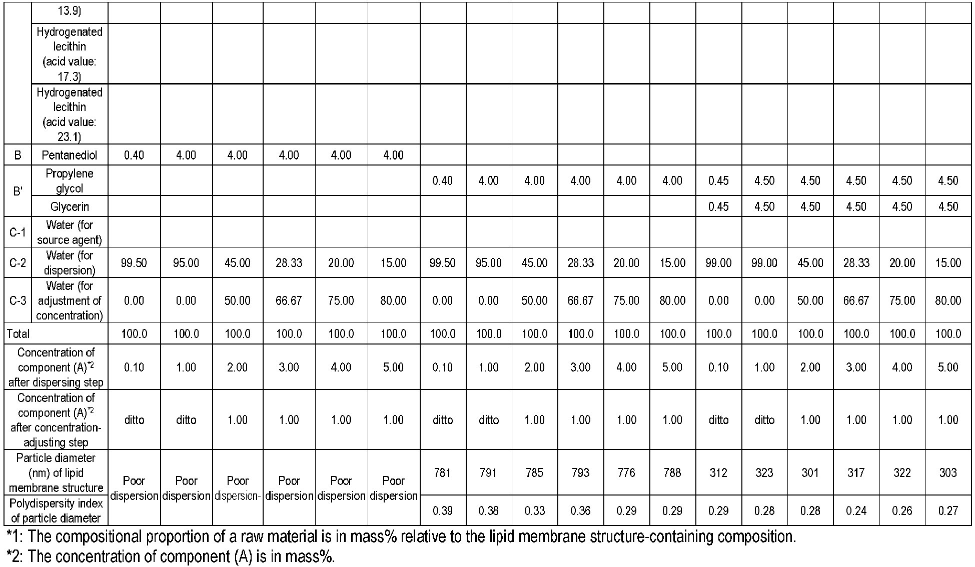

As shown in Table 1, it was demonstrated that the particle diameter of a resulting lipid membrane structure increases as the concentration of the component (A) in the dispersion is increased in the dispersing step depending on the target particle diameter of the lipid membrane structure included in the lipid membrane structure-containing composition. In contrast, as shown in Table 2, it was demonstrated that when the acid value of the hydrogenated phospholipid is less than 5 mg KOH/g or when propylene glycol or glycerin is included instead of the component (B) in the lipid membrane structure-containing composition, the particle diameter of the resulting lipid membrane structure is not consistent with the relationship of the present invention or a dispersion cannot be prepared (aggregation occurs due to poor dispersibility), even if the concentration of the dispersion in the dispersing step is changed.

Regarding each of the series of Examples 1-0 to 1-5, Examples 2-0 to 2-5, and Examples 3-0 to 3-5, the concentration [%] of the component (A) in the dispersion obtained in the dispersing step is defined as an explanatory variable (x), and the particle diameter [nm] of the lipid membrane structure included in the finally obtained lipid membrane structure-containing composition is defined as a dependent variable (y). On this occasion, the approximate curve (3rd degree polynomial) and the value of its coefficient of determination (R²) acquired using the function of the "polynomial approximation (3rd degree)" in the "approximate curve formatting" of Microsoft Excel (produced by Microsoft Corporation) are shown in Table 3 below.

**[Table 3]**

| | Approximate curve (3rd degree polynomial) | Coefficient of determination R² |
|---|---|---|
| Example 1 series | y = -186470x³ + 26536x² + 101.74x + 80.77 | 0.9998 |
| Example 2 series | y = -13263x³ + 16590x² + 126.93x + 81.926 | 0.9999 |
| Example 3 series | y = 846175x³ - 27979x² + 474.32x + 87.483 | 0.9998 |

From the above, it was demonstrated that the particle diameter of a resulting lipid membrane structure can be controlled by combining a hydrogenated phospholipid having an acid value of 5 mg KOH/g or more and a polyol compound having a specific structure and determining the concentration of the component (A) in the dispersion in the dispersing step depending on the target particle diameter of the lipid membrane structure included in the lipid membrane structure-containing composition.

The present application is based on Japanese Patent Application No. 2022-140891, filed on September 5, 2022, the disclosure of which is incorporated herein by reference in its entirety.

## Claims

1. A method for producing a lipid membrane structure-containing composition comprising:
(A) a hydrogenated phospholipid having an acid value of 5 mg KOH/g or more;
(B) a compound represented by a following formula 1: 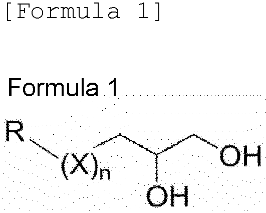 wherein
R is a substituted or unsubstituted alkyl group having 2 to 6 carbon atoms or a substituted or unsubstituted cycloalkyl group having 3 to 6 carbon atoms,
X is -O-, -C(=O)O-, or -O-C(=O)-, and
n is 0 or 1; and
(C) water;
the method comprising:
a dispersing step of mixing the component (A), the component (B), and the component (C) to obtain a dispersion in which a lipid membrane structure having a particle diameter of 10 nm or more and 200 nm or less is spontaneously formed, wherein
the dispersing step includes determining a concentration of the component (A) in the dispersion depending on a target particle diameter of the lipid membrane structure included in the lipid membrane structure-containing composition.

2. The method according to Claim 1, wherein a content of the component (B) in the dispersion is more than 100 parts by mass per 100 parts by mass of the component (A).

3. The method according to Claim 1 or 2, wherein a concentration of the component (A) in the dispersion is from 0.01 to 15 mass%.

4. The method according to Claim 1 or 2, further comprising a concentration-adjusting step of mixing the dispersion and the component (C) to obtain the lipid membrane structure-containing composition.

5. The method according to Claim 1 or 2, wherein in the dispersing step, the component (A), the component (B), and the component (C) are mixed at a phase transition temperature of the component (A) or higher.

6. A method for producing a cosmetic, comprising producing a lipid membrane structure-containing composition by the method according to Claim 1 or 2.

7. A method for producing an external preparation for the skin, comprising producing a lipid membrane structure-containing composition by the method according to Claim 1 or 2.
